(19) Europäisches Patentamt
European Patent Office
Office européen des brevets

(11) Veröffentlichungsnummer: **0 053 775**
**B1**

(12) # EUROPÄISCHE PATENTSCHRIFT

(45) Veröffentlichungstag der Patentschrift: **07.05.86**

(21) Anmeldenummer: **81109985.2**

(22) Anmeldetag: **28.11.81**

(51) Int. Cl.$^4$: **C 07 D 401/12,**
C 07 D 401/14,
C 07 D 251/18,
C 07 D 251/50,
C 07 D 251/52,
C 07 D 251/70, C 08 K 5/34

(54) **Poly-bis-triazinylamine, Verfahren zu ihrer Herstellung, ihre Verwendung zum Stabilisieren von synthetischen Polymeren und die so stabilisierten Polymeren.**

(30) Priorität: **05.12.80 DE 3045839**

(43) Veröffentlichungstag der Anmeldung:
**16.06.82 Patentblatt 82/24**

(45) Bekanntmachung des Hinweises auf die Patenterteilung:
**07.05.86 Patentblatt 86/19**

(84) Benannte Vertragsstaaten:
**AT BE CH DE FR GB IT LI NL SE**

(56) Entgegenhaltungen:
**EP-A-0 002 754**
**EP-A-0 013 665**
**EP-A-0 014 683**
**EP-A-0 024 338**
**EP-A-0 044 499**
**DE-A-2 636 144**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Wiezer, Hartmut, Dr.**
**Hans-Fischer-Strasse 6**
**D-8906 Gersthofen (DE)**
Erfinder: **Pfahler, Gerhard, Dr.**
**Karlsbader Strasse 27**
**D-8900 Augsburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 053 775 B1

**Beschreibung**

Die Erfindung betrifft neue Poly-bis-triazinylamine, ein Verfahren zu ihrer Herstellung sowie ihre Verwendung als Lichtschutzmittel mit antioxidativer Wirkung für synthetische Polymere.

Die neuen Poly-bis-triazinylamine werden durch die allgemeine Formel (I)

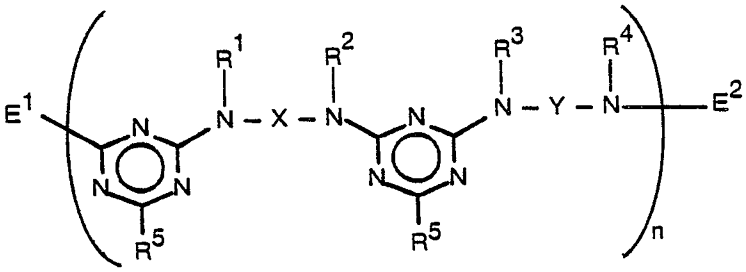

(I)

charakterisiert. In dieser Formel bedeutet:

n eine ganze Zahl von 1 bis 200, vorzugsweise von 1 bis 50 und insbesondere von 1 bis 15.

$R^1$ und $R^2$ sind gleich oder verschieden und stehen für Wasserstoff, für unsubstituiertes oder durch eine Hydroxylgruppe substituiertes $C_1$- bis $C_{37}$-, vorzugsweise $C_1$- bis $C_{18}$ und insbesondere $C_1$- bis $C_{10}$-Alkyl, oder für unsubstituiertes oder durch bis zu vier $C_1$- bis $C_4$-Alkylgruppen substituiertes, vorzungsweise unsubstituiertes $C_5$- bis $C_{12}$-Cycloalkyl, oder für $C_7$- bis $C_{14}$-Aralkyl, vorzugsweise $C_7$- bis $C_9$-Phenylalkyl, oder eine Gruppe der Formel (II)

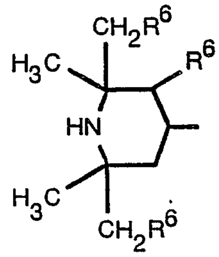

(II),

in welcher $R^6$ Wasserstoff oder $C_1$- bis $C_6$-Alkyl, vorzugsweise Wasserstoff oder Methyl, insbesondere aber Wasserstoff, bedeutet.

$R^3$ und $R^4$ sind gleich oder verschieden und haben die für $R^1$ und $R^2$ angegebenen Bedeutungen. Vorzugsweise ist einer dieser Reste Wasserstoff, insbesondere sind es jedoch beide.

$R^5$ ist Halogen, Phenyl, —$OR^7$ oder —$NR^8R^9$ und insbesondere —$NR^8R^9$, wobei in dieser Gruppen $R^7$ die Bedeutung von Wasserstoff, von $C_1$ bis $C_{18}$-, vorzugsweise $C_1$- bis $C_4$-Alkyl, Von $C_3$- bis $C_{12}$-Alkenyl, von $C_5$- bis $C_{12}$-Cycloalkyl, von $C_7$- bis $C_{18}$-Aralkyl, von $C_6$- bis $C_{10}$-Aryl oder eines Rests der Formel (II) hat. Bevorzugte Reste $R^7$ sind $C_1$- bis $C_4$-Alkyl, Phenyl ode der Rest der Formel (II).

$R^8$ und $R^9$ sind gleich oder verschieden und stehen für Wasserstoff, für $C_1$- bis $C_{37}$-Alkyl, das insbesondere bei $C_2$- und $C_3$-Alkyl durch $C_1$- bis $C_{18}$-Alkoxy und bei $C_2$- bis $C_5$-Alkyl durch $C_1$- bis $C_4$-, vorzugsweise $C_1$- bis $C_2$-Di-alkylamino, substituiert sein kann, für $C_3$- bis $C_{12}$-Alkenyl, für unsubstituiertes oder durch bis zu vier $C_1$- bis $C_4$-Alkylgruppen substituiertes $C_5$- bis $C_{12}$-Cycloalkyl, für $C_7$- bis $C_{18}$-Aralkyl, vorzugsweise $C_7$ bis $C_9$-Phenylalkyl, für Phenyl oder Naphthyl, vorzugsweise Phenyl, für $C_2$- bis $C_{30}$-, vorzugsweise $C_2$- bis $C_{18}$- und insbesondere $C_2$- bis $C_4$-Hydroxialkyl oder für den Rest der Formel (II).

$R^8$ und $R^9$ können auch zusammen mit dem gemeinsamen Stickstoffatom einen Pyrrolidin- oder einen unsubstituierten oder durch bis zu vier $C_1$- bis $C_4$-Alkylgruppen substituierten Piperidin-, Morpholin- oder Hexamethyleniminring bilden.

X und Y sind gleich oder verschieden und bedeuten $C_2$- bis $C_{18}$-, vorzugsweise $C_2$- bis $C_{12}$- und insbesondere $C_2$- bis $C_6$-Alkylen, oder $C_2$- bis $C_{12}$-Bis-(propyloxi)-alkylen, oder $C_6$- bis $C_{18}$, vorzugsweise $C_6$- bis $C_{12}$-unsubstituiertes oder durch bis zu vier Methylgruppen substituiertes, vorzugsweise jedoch unsubstituiertes Mono-, Di- oder Tricycloalkylen, in welchem bei dem erstgenannten zwei C-Atome durch N-Atome, welche Propylengruppe tragen können, ersetzt sein können, oder $C_6$- bis $C_{18}$-Arylen, vorzugsweise Phenylen, oder $C_7$- bis $C_{18}$-Aralkylen, insbesondere X und Y aber Di- oder Tricycloalkylen sind, oder auch anstelle der Gruppierungen

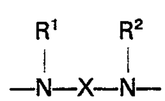

oder

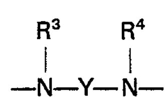

Piperazinylen-Reste treten.

$E^1$ ist ein Halogenatom oder eine Gruppe

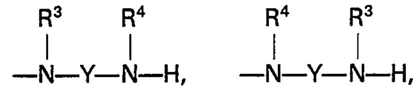

—$OR^7$ oder —$NR^8R^9$, vorzugsweise Chlor und

$E_7$ ist Wasserstoff oder eine Acylgruppe, vorzugsweise Wasserstoff, wobei $R^3$, $R^4$, $R^7$, $R^8$ und $R^9$ die oben angegebenen Bedeutungen haben und mindestens einer der Reste $R^1$, $R^2$, $R^3$, $R^4$, $R^7$, $R^8$ und $R^9$ eine Gruppe der Formel (II) sein muß, mit der Bedingung, daß $R^1$ bis $R^4$ gleich sind, wenn X und Y verschieden sind, und X und Y gleich sind, wenn mindestens einer der Reste $R^1$ oder $R^2$ von $R^3$ und $R^4$ verschieden ist.

Die neuen Poly-bis-triazinylamine werden ausgehend von un- oder teilsubstituierten Cyanurhalogeniden erhalten. Die einzelnen Zwischenprodukte können isoliert werden, jedoch ist es auch möglich, die Synthese der gewünschten Endprodukte in einem sogenannten "Eintopfverfahren" ohne Isolierung der Zwischenverbindungen zu vollziehen.

Zur Durchführung der Synthese bieten sich zwei Reaktionswege an, die im nachfolgenden, vereinfachten Reaktionsschema skizziert sind.

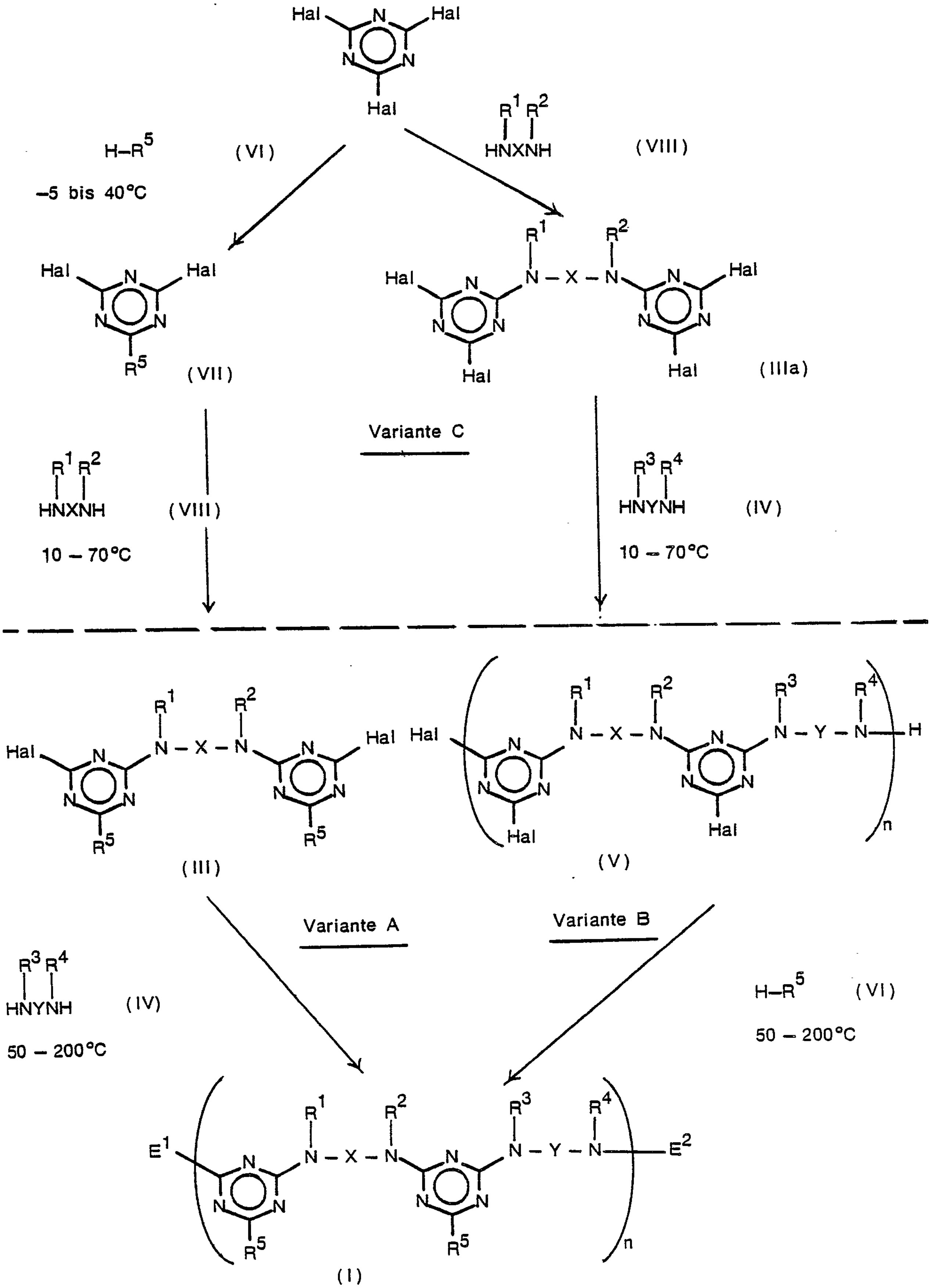
(VI)
H–R5
−5 bis 40°C
(VIII)
HNXNH
(VII)
(IIIa)
Variante C
(VIII)
HNXNH
10 – 70°C
(IV)
HNYNH
10 – 70°C
(III)
(V)
Variante A
Variante B
(IV)
HNYNH
50 – 200°C
(VI)
H–R5
50 – 200°C
(I)

Die Reihenfolge, in der die Diamine (IV) und (VIII) eingesetzt werden, ist beliebig, die im Schema darelegte Reihenfolge stellt jedoch die bevorzugte Arbeitsweise dar. In den Formeln des Reaktionsschemas haben Hal, n, $R^1$, $R^2$, $R^3$, $R^4$, $R^5$, X, Y, $E^1$ und $E^2$ die oben angegebenen Bedeutungen. In Formel (VI) kann $R^5$ nicht Halogen oder Phenyl sein.

Nach Variante A wird ein Dihalogen-bis-triazinyldiamin der Formel (III) mit der 0,8- bis 1,4-, vorzugsweise der 1,0- bis 1,2- und insbesondere der 1- bis 1,05fach molaren Menge eines Diamins der Formel (IV) umgesetzt.

Variante B beinhaltet die Reaktion eines Poly-bis-triazinylamins der Formel (V), welches aus den Verbindungen (IIIa) durch Umsetzen mit der 0,8- bis 1,4-, vorzugsweise der 1,0- bis 1,2- und insbesondere der 1- bis 1,05fach molaren Menge eines Diamins der Formel (IV) zugänglich ist, mit der 0,8- bis 1,4-, vorzugsweise der 1,0- bis 1,2- und insbesondere der 1,0- bis 1,05fach valenten Menge einer Verbindung der Formel (VI).

Variante C ist ein Mehrstufen-Eintopfverfahren, bei dem zunächst Cyanurhalogenid, vorzugsweise Cyanurchlorid, zur Synthese der Zwischenverbindungen (VII) mit der 0,8- bis 1,3-, vorzugsweise der 0,9- bis 1,1- und insbesondere der äquimolaren Menge einer Verbindung der Formel (VI) oder zur Herstellung der Zwischenverbindungen (IIIa) mit der 0,4- bis 0,7-, vorzugsweise der 0,5- bis 0,6- und insbesondere der 0,5- bis 0.502fach molaren Menge eines Diamins der Formel (VIII) umgesetzt wird. Die so erhaltenen Zwischenverbindungen setzt man sodann ohne Isolierung mit der 0,4- bis 0,7-, vorzugsweise der 0,5- bis 0,6- und insbesondere der 0,5- bis 0,502fach molaren Menge entweder eines Diamins der Formel (VIII), wenn eine Verbindung der Formel (VII) Edukt ist, oder der 0,8- bis 1,4-, vorzugsweise der 1,0 bis 1,2- und insbesondere der 1,0-bis 1,05fach molaren Menge eines Diamins der Formel (IV), wenn eine Verbindung der Formel (IIIa) Edukt ist, um. Im Anschluß daran werden die auf diese Weise dargestellten Zwischenverbindungen (III) bzw. (V), ebenfalls ohne Zwischenisolierung, unter den für die Varianten A bzw. B angegebenen Bedingungen zu dem gewünschten Poly-bis-triazinylamin umgesetzt.

Eine bevorzugte Ausführungsform ist die Variante A und die über diese Durchführungsform als Zwischenstufe ablaufende "Mehrstufen-Eintopfreaktion".

Die Reaktionen werden in inerten organischen Lösungsmitteln wie z.B. Petrolether, Benzinen, Aceton, Ether, Dioxan, Benzol, Toluol, Xylol, Cumol, Mesitylen oder Gemischen derselben vorgenommen.

Die Varianten A und B erfordern Reaktionstemperaturen von 50 bis 200, vorzugsweise 80 bis 180 und insbesondere 100 bis 160°C. Nach dem Verfahren gemäß Variante C wird die Monosubstitution des Cyanurhalogenids zu Verbindung (VII) bei −5 bis 40, vorzugsweise −5 bis 20 und insbesondere 0 bis 10°C, die Monosubstitution des Cyanurhalogenids zu Verbindung (IIIa) bei −5 bis 20, vorzugsweise bei 0 bis 10°C, die Disubstitution des Dihalogentriazins (VII) zu (III) und die des Tetrahalogenditriazins (IIIa) zu (V) bei 10 bis 70, vorzugsweise 30 bis 70 und insbesondere 40 bis 60°C durchgeführt.

Bei der Herstellung der neuen Verbindungen gibt man als Halogenwasserstoffakzeptoren anorganische Basen in äquivalenten Mengen zu. Geeignet sind z.B. Natriumhydroxid, Kaliumhydroxid, Natriumcarbonat und Kaliumcarbonat in fester Form oder wäßriger Lösung.

Beispiele für Dihalogentriazinzwischenverbindungen der Formel (VII) sind:

2,4-Dichlor-6-[N-(2,2,6,6-tetramethyl-4-piperidyl)-3-hydroxipropylamino]-1,3,5-triazin
2,4-Dichlor-6-[N-(2,2,6,6-tetramethyl-4-piperidyl)-2-methoxipropylamino]-1,3,5-triazin
2,4-Dichlor-6-[N-(2,2,6,6-tetramethyl-4-piperidyl)-3-ethoxipropylamino]-1,3,5-triazin
2,4-Dichlor-6-[N-(2,2,6,6-tetramethyl-4-piperidyl)-3-octadecyloxipropylamino]-1,3,5-triazin
2,4-Dichlor-6-[N-(2,2,6,6-tetramethyl-4-piperidyl)-3-dimethylaminopropylamino]-1,3,5-triazin
2,4-Dichlor-6-[N-(2,2,6,6-tetramethyl-4-piperidyl)-3-diethylaminopropylamino]-1,3,5-triazin
2,4-Dichlor-6-[N-(2,2,6,6-tetramethyl-4-piperidyl)-2-dimethylaminoethylamino]-1,3,5-triazin
2,4-Dichlor-6-[N-(2,2,6,6-tetramethyl-4-piperidyl)-4-diethylaminobutylamino]-1,3,5-triazin
2,4-Dichlor-6-[N-(2,2,6,6-tetramethyl-4-piperidyl)-4-diethylamino-1-methylbutylamino]-1,3,5-triazin
2,4-Dichlor-6-dioctadecylamino-1,3,5-triazin
2,4-Dichlor-6-butylamino-1,3,5-triazin
2,4-Dichlor-6-octadecylamino-1,3,5-triazin
2,4-Dichlor-6-dicyclohexylamino-1,3,5-triazin
2,4-Dichlor-6-dibutylamino-1,3,5-triazin
2,4-Dichlor-6-amino-1,3,5-triazin
2,4-Dichlor-6-[N-(2,2,6,6-tetramethyl-4-piperidyl)-amino]-1,3,5-triazin
2,4-Dichlor-6-[N-(2,2,6,6-tetramethyl-4-piperidyl)-butylamino]-1,3,5-triazin
2,4-Dichlor-6-[N-(2,2,6,6-tetramethyl-4-piperidyl)-hexylamino]-1,3,5-triazin
2,4-Dichlor-6-[N-(2,2,6,6-tetramethyl-4-piperidyl)-octadecylamino]-1,3,5-triazin
2,4-Dichlor-6-[N-bis-(2,2,6,6-tetramethyl-4-piperidyl)-amino]-1,3,5-triazin
2,4-Dichlor-6-(1,1,3,3-tetramethyl)-butylamino-1,3,5-triazin
2,4-Dichlor-6-morpholino-1,3,5-triazin
2,4-Dichlor-6-phenoxi-1,3,5-triazin.

Beispiele für Verbindungen der Formeln (VIII) und (IV) sind:

Ethylendiamin, Propylendiamin, 1,6-Diaminohexan, 1,8-Diaminooctan, 1,12-Diaminododecan, 1,2-Diaminopropan, 2,5-Diamino-2,5-dimethylhexan, p-Phenylen-diamin, 1,8-Diamino-p-menthan, 1,3-Bis-(aminomethyl)-cyclohexan, 4,4'-Diaminodicyclohexylmethan, 3,3'-Dimethyl-4,4'-diaminodicyclohexylmethan, 4,4'-Diaminodiphenylmethan, 2 (3), 5 (6)-Bis-(amino-methyl)-norbornan-Isomerengemisch, 3 (4), 8 (9)-Bis-(aminomethyl)-tricyclo-[5.2.1.$0^{2,6}$] decan-Isomerengemisch, Bis-(3-aminopropyl)-piperazin, 4,7-Dioxadecan-1,10-diamin, 4,9-Dioxadodecan-1,12-diamin, 7-Methyl-4,10-dioxatridecan-1,13-diamin; 3-Amino-1-methylaminopropan, 3-Amino-1-cyclohexyl-aminopropan, 3-Amino-1-(2-ethyl)-hexylamino-propan, N-(Cyclohexyl)-ethylendiamin, N-(Cyclohexyl)-1,6-diaminohexan, N-(2-Hydroxipropyl)-ethylendiamin,
N-(2,2,6,6-Tetramethyl-4-piperidyl)-ethylendiamin
N-(2,2,6,6-Tetramethyl-4-piperidyl)-1,6-diaminohexan
N-(2,2,6,6-Tetramethyl-4-piperidyl)-1,3-bis-(aminomethyl)-cyclohexan,
N-(2,2,6,6-Tetramethyl-4-piperidyl)-3(4), 8(9)-bis-(aminomethyl)-tricyclo-[5.4.1.$0^{2,6}$]-decan-Isomerengemisch;
N,N'-Bis-(2,2,6,6-tetramethyl-4-piperidyl)-ethylendiamin
N,N'-Bis-(2,2,6,6-tetramethyl-4-piperidyl)-1,6-diaminohexan
N,N'-Bis-(2,2,6,6-tetramethyl-4-piperidyl)-3(4), 8(9)-bis-(aminomethyl)-tricyclo-[5.4.1.$0^{2,6}$]-decan-Isomerengemisch;
N,N'-Bis-(2,2,6,6-tetramethyl-4-piperidyl)-1,3-bis-(aminomethyl)cyclohexan,
N,N'-Bis-(2,2,6,6-tetramethyl-4-piperidyl)-4,9-dioxadodecan-1,12-diamin,
N,N'-Bis-cyclododecyl-1,6-diaminohexan,
N,N'-bis-isopropyl-1,6-diaminohexan,
N,N'-bis-cyclohexyl-1,6-diaminohexan,
N,N'-Bis-(2,2,6,6-tetramethyl-4-piperidyl)-4,4'-diamino-dicyclohexylmethan,
N-(2,2,6,6-Tetramethyl-4-piperidyl)-N'-(cyclododecyl)-1,6-diaminohexan,
Piperazin.

Beispiele für Verbindungen der Formel (VI) sind:
N-(2,2,6,6-Tetramethyl-4-piperidyl)-3-methoxipropylamin
N-(2,2,6,6-Tetramethyl-4-piperidyl)-3-ethoxipropylamin
N-(2,2,6,6-Tetramethyl-4-piperidyl)-3-octadecyloxipropylamin
N-(2,2,6,6-Tetramethyl-4-piperidyl)-3-dimethylaminopropylamin
N-(2,2,6,6-Tetramethyl-4-piperidyl)-2-dimethylaminoethylamin
N-(2,2,6,6-Tetramethyl-4-piperidyl)-4-diethylaminobutylamin
2,2,6,6-Tetramethyl-4-piperidylaminopropanol-3
N-(2,2,6,6-Tetramethyl-4-piperidyl)-4-diethylamino-1-methylbutylamin
2,2,6,6-Tetramethyl-4-aminopiperidin
N-(2,2,6,6-Tetramethyl-4-piperidyl)-butylamin
N-(2,2,6,6-Tetramethyl-4-piperidyl)-octadecylamin
N-(2,2,6,6-Tetramethyl-4-piperidyl)-cyclododecylamin
N-(2,2,6,6-Tetramethyl-4-piperidyl)-hexylamin
Di-(2,2,6,6-Tetramethylpiperidyl-4)-amin,
Ammoniak, Butylamin, Dicyclohexylamin, Cyclododecylamin, Hexylamin, Dodecylamin, Octadecylamin, Dioctadecylamin, 2,3-Dimethylcyclohexylamin, 2,6-Dimethylmorpholin, Methylcyclohexylamin, Pyrrolidin, Piperidin, 3,5,5-Trimethylhexylamin, 2,2-Dimethylpropylamin, Di-n-octylamin, Methanol, Phenol, 2,2,6,6-Tetramethylpiperidinol-4.

Beispiele für Verbindungen der Formel (III) sind:
1 N,N'-Bis-[2-Chlor-4-<(2,2,6,6-tetramethyl-4-piperidyl)-3-methoxipropylamino>-1,3,5-triazin-6-yl]-N-(2,2,6,6-tetramethyl-4-piperidyl)-1,6-diaminohexan
2 N,N'-Bis-[2-Chlor-4-<(2,2,6,6-tetramethyl-4-piperidyl)-3-methoxipropylamino>-1,3,5-triazin-6-yl]-N-(2,2,6,6-tetramethyl-4-piperidyl)-1,3-bis-(aminomethyl)-cyclohexan
3 N,N'-Bis-[2-Chlor-4-<(2,2,6,6-tetramethyl-4-piperidyl)-3-methoxipropylamino>-1,3,5-triazin-6-yl]-N,N'-bis-(2,2,6,6-tetramethyl-4-piperidyl)-3(4), 8(9)-bis-(aminomethyl)-tricyclo-[5.2.1.$0^{2,6}$]-decan-Isomerengemisch
4 N,N'-Bis-[2-Chlor-4-<(2,2,6,6-tetramethyl-4-piperidyl)-3-ethoxipropylamino>-1,3,5-triazin-6-yl]-N-(2,2,6,6-tetramethyl-4-piperidyl)-4,9-dioxadodecan-1,12-diamin
5 N,N'-Bis-[2-Chlor-4-<(2,2,6,6-tetramethyl-4-piperidyl)-butylamino>-1,3,5-triazin-6-yl]-N,N'-bis-(2,2,6,6-tetramethyl-4-piperidyl)-1,6-diaminohexan
6 N,N'-Bis-[2-Chlor-4-<(2,2,6,6-tetramethyl-4-piperidyl)-butylamino>-1,3,5-triazin-6-yl]-N,N'-bis-cyclododecyl-1,6-diaminohexan
7 N,N'-Bis-[2-Chlor-4-<(2,2,6,6-tetramethyl-4-piperidyl)-butylamino>-1,3,5-triazin-6-yl]-N-cyclohexyl-1,3-diaminopropan
8 N,N'-Bis-[2-Chlor-4-dibutylamino-1,3,5-triazin-6-yl]-N,N'-bis-(2,2,6,6-tetramethyl-4-piperidyl)-ethylendiamin
9 N,N'-Bis-[2-Chlor-4-morpholino-1,3,5-triazin-6-yl]-N,N'-bis-(2,2,6,6-tetramethyl-4-piperidyl)-ethylendiamin
10 N,N'-Bis-[2-Chlor-4-octadecylamino-1,3,5-triazin-6-yl]-N,N'-bis-(2,2,6,6-tetramethyl-4-piperidyl)-ethylendiamin
11 N,N'-Bis-[2-Chlor-4-<(2,2,6,6-tetramethyl-4-piperidyl)-octadecylamino>-1,3,5-triazin-6-yl]-piperazin

12 N,N'-Bis-[2-Chlor-4-<(2,2,6,6-tetramethyl-4-piperidyl)-3-ethoxipropylamino>-1,3,5-triazin-6-yl]-bis-(3-aminopropyl)-piperazin

13 N,N'-Bis-[2-Chlor-4-phenoxi-1,3,5-triazin-6-yl]-N,N'-bis-(2,2,6,6-tetramethyl-4-piperidyl)-ethylendiamin

14 N,N'-Bis-[2-Chlor-4-phenyl-1,3,5-triazin-6-yl]-N,N'-bis-(2,2,6,6-tetramethyl-4-piperidyl)-4,9-dioxadodecandiamin-1,12

Die Umsetzung von Cyanurhalogeniden mit primären oder sekundären Mono- oder Polyaminen und auch Alkoholen oder Phenolen zu definierten Verbindungen oder polymeren Triazinen ist seit geraumer Zeit Stand der Technik [J. Am. Chem. Soc. 73 (1951), Nr. 7, Seiten 2981 ff; US—PS 2 544 071- CH—PS 32 784 und 342 785].

Bekannt sind ferner Polytriazinverbindungen der Formel

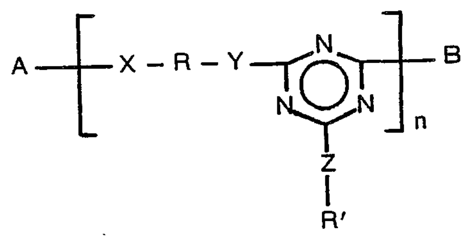

worin X und Y und Z gleich oder verschieden sein können und —O—, —S— oder

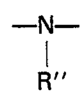

bedeuten und eine dieser Gruppen einen Polyalkylpiperidylrest tragen muß (DE—PS 26 36 144). Wie bereits in der Europäischen Patentanmeldung 13 665, welche ebenfalls polymere, der obigen Patentschrift verwandte Produkte beschreibt, dargelegt wird, hat sich gezeigt, daß bei der Herstellung dieser Produkte erhebliche Schwierigkeiten in Kauf genommen werden müssen, die im wesentlichen auf die Bildung unlöslicher, schwer entfernbarer Nebenprodukte zurückzuführen sind. Ein weiterer Nachteil ist darin zu sehen, daß im Endprodukt ein merklicher Anteil an niedrigmolekularen Bestandteilen enthalten ist, was eine relativ hohe Flüchtigkeit bewirkt. Zusammenfassend gesagt, sind die Nachteile der in der DE—PS 26 36 144 beschriebenen Produkte in einer zu breiten Molekulargewichtsverteilung zu sehen: Zu hochmolekulare Anteile verursachen die Unlöslichkeit auch im Polymeren, zu niedrigmolekulare ein Ansteigen der Flüchtigkeit. Die Nachteile können nur durch aufwendige Trenn- und Reinigungsoperationen beseitigt werden. Nachteilig bei den Produkten der EP—A 13 665 ist, daß in hohem Maße vernetzte Produkte entstehen können, die dann Verträglichkeitsprobleme mit den zu stabilisierenden Polymeren aufwerfen.

Daß von den erfindungsgemäßen Verbindungen auch die, in denen $R^8$ oder $R^9$ die Bedeutung einer Hydroxialkyl-, Alkoxipropyl- oder Dialkylaminoalkylgruppe hat, herstellbar sind, war keineswegs vorhersehbar, denn diese Gruppen können analog der NH-Gruppe ebenfalls mit Säurechloriden reagieren, und es war daher damit zu rechnen, daß es zu Brückenbildungen zwischen zwei verschiedenen Triazinringen kommen würde, was zu unerwünschten vernetzten Produkten geführt hätte. Veröffentlichungen über die bekannte Bildung von Cyanursäureestern aus Alkoholen, welche auch durch Etherspaltung aufgrund nicht auszuschließender Mengen Halogenwasserstoff aus Alkoxialkylgruppen entstehen konnen, sowie über die Synthese von Cyanursäuredialkylamiden aus Cyanurchlorid und tert. Aminen ließen einen derartigen Reaktionsverlauf als durchaus möglich, wenn nicht sogar bevorzugt erscheinen [Ullmann Bd. 9 (1975), S. 651; E. Kober und R. Rätz, J. Org. Chem. 27 (1962), S. 2509 ff].

Mit den erfindungsgemäßen Poly-bis-triazinylaminen ist die der vorliegenden Erfindung zugrunde liegende Aufgabe, d.h. linear aufgebaute, nicht vernetzte polymer Lichtschutzmittel bereitzustellen, die eine ausgezeichnete Lichtschutzwirkung aufweisen und von den Nachteilen der Produkte der DE-PS 26 36 144 frei sind, zu erfüllen, denn die neuen Verbindungen besitzen diese angestrebten Eigenschaften in hohem Maße, was sich keineswegs erwarten ließ. Es konnte nämlich nicht vorhergesehen werden, daß die Maßnahme, zunächst Dichlor-bis-triazinylamine (III) herzustellen und diese gezielt mit ausgewählten anderen Diaminen (IV) umzusetzen, zu Polymeren führen würde, die dem gewünschten Eigenschaftsbild entsprochen hätten. Man hätte vielmehr annehmen müssen, daß die neuen Produkte in ihren Eigenschaften denen der DE—PS 26 36 144 weitgehend ähnlich sein würden. Überraschenderweise kann man jedoch durch gezielte Auswahl der Diamine (IV) die Eigenschaften der neuen erfindungsgemäßen Polytriazine steuern, damit z.B. das Molgewicht weitestgehend beeinflussen und bei hohem Anteil an Gruppen der Formel (II) auch in kurzer Reaktionszeit hohe Molgewichte erzielen.

Neben ihrer ausgezeichneten Wirksamkeit ist bei den neuen Produkten auf die im Vergleich zu dem besten Beispiel der genannten DE—PS 26 36 144, Bsp. 6, hervorstechande niedrige Flüchtigkeit hinzuweisen, was sich besonders stark bei denen, in welchen die Reste $R^3$ und/oder $R^4$ = Wasserstoff sind, äußert.

Die neuen Triazinstabilisatoren lassen sich problemlos in die zu stabilisierenden Polymeren cinarbeiten und sind hervorragend zum Stabilisieren derselben gegen den lichtinduzierten oxidativen Abbau geeignet. Neben der ausgezeichneten Stabilisatorwirksamkeit zeichnet die neuen Stabilisatoren ihre Verträglichkeit mit den zu stabilisierenden Polymeren, ihre Migrationsfestigkeit gegen das Auswaschen mit wäßrigen Medien, welche bei der Freibewitterung eine bedeutende Rolle spielt, ihre Thermostabilität auch bei hohen Verarbeitungstemperaturen und ihre geringe Flüchtigkeit, insbesondere im Vergleich zu Beispiel 6 der DE—PS 26 36 144 aus.

Die neuen Verbindungen werden — wie bereits ausgeführt — als Stabilisatoren für Kunststoffe gegen deren Schädigung durch Einwirkung von Sauerstoff, Wärme und Licht verwendet, Beispiele für solche Kunststoffe sind im einzelnen:

Polymere, die sich von einfach oder doppelt ungesättigten Kohlenwasserstoffen ableiten, z.B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrunde liegenden Monomeren, wie Ethylen-Propylen-Copolymere, Propylen-Buten-1-Copolymere, Propylen-Isobuten-Copolymere, Styrol-Butadien-Copolymere sowie Terpolymere von Ethylen und Propylen mit einem Dien, wie z.B. Hexadien, Dicyclopentadien oder Ethylidennorbornen; Mischungen der obengenannten Homopolymeren, wie beispielsweise Gemische von Polypropylen und Polyethylen, Polypropylen und Polybuten-1, Polypropylen und Polyisobutylen oder von Butadien-Acrylnitril-Copolymerisat mit einem Styrol-Butadien-Copolymerisat.

Halogenhaltige Vinylpolymere, wie Polyvinylchlorid, Polyvinylidenchlorid, Polyvinylfluorid, Polychloropren und Chlorkautschuke sowie Copolymere von Vinylchlorid und Vinylidenchlorid untereinander und mit anderen olefinisch ungesättigten Monomeren.

Polymere, die sich von α,β-ungesättigten Säuren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril-Butadien-Styrol-, Acrylnitril-Styrol- und Acrylnitril-Styrol-Acrylester-Copolymerisate.

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen-Vinylacetat-Copolymere.

Homo- und Copolymere, die sich von Epoxiden ableiten, wie Polyethylenoxid oder die Polymerisate, die sich von Bisglycidylethern ableiten.

Polyacetale, wie Polyoximethylen und Polyoxiethylen sowie solche Polyoximethylene, die als Comonomeres Ethylenoxid enthalten.

Polyurethane und Polyharnstoffe

Polycarbonat

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxicarbonsäuren oder den entsprechenden Lactonen ableiten, wie Polyethylenterephthalat, Polybutylenterephthalat, Poly-1,4-dimethylol-cyclohexanterephthalat.

Vernetzte Polymerisate, die sich von Aldehyden einerseits und Phenolen, Harnstoffen und Melaminen andererseits ableiten, wie Phenol-Formaldehyd;, Harnstoff-Formaldehyd- und Melamin-Formaldehydharze.

Die neuen Verbindungen lassen sich schließlich auch als Stabilisatoren auf dem Harz- und Lacksektor einsetzen. Beispiele sind duroplastische und thermoplastische Acrylharze, welche für Autolackierungen verwendet werden [Encyclopedia of Polymer Science and Technology, Interscience Publishers, New York, Band 1 (1964), Seiten 273—276, und Band 13 (1970), Seiten 530—532; "Understanding Paint" von W. R. Fuller, American Paint Journal Co., St. Louis, 1965, Seiten 124—135], Acrylharzlacke, das sind die üblichen Einbrennlacke (beschrieben z.B. in H. Kittel's "Lehrbuch der Lacke und Beschichtungen", Band 1, Teil 2, Seite 735 und 742 (Berlin, 1972) und in H. Wagner, H. F. Sarx "Lackkunstharze", Seiten 229—235] sowie ganz besonders Mischungen auf der Basis von heiß-vernetzbarem Acrylharz und Styrol sowie Lacke und Beschichtungen auf der Basis von Acryl/Melaminharz und Alkyd/Acryl/Melaminharz. Derartige Lacke können als weitere Zusatzstoffe andere übliche Lichtschutzmittel, phenolische Antioxidantien, Pigmente, Farbstoffe, Metalldesaktivatoren etc. enthalten.

Von besonderer Bedeutung ist die Stabilisierung von Polyolefinen, Styrolpolymerisaten, Polyamiden, Poly-(meth)acrylaten und von Polyurethanen, für die sich die Verbindungen bevorzugt eignen. Beispiele hierfür sind Polyethylen hoher und niedriger Dichte, Polypropylen, Ethylen-Propylen-Copolymerisate, Polystyrol, Styrol-Butadien-Acrylnitril-Terpolymerisate; Mischungen von Polyolefinen oder von Styrolpolymerisaten sowie Polyurethane auf Polyether- oder Polyesterbasis.

Die neuen Stabilisatoren werden nach allgemein üblichen Methoden in die Polymermassen eingearbeitet. Die Einarbeitung kann beispielsweise durch Einmischen der Verbindungen und gegebenenfalls weiterer Additive in die Schmelze nach dan in der Technik üblichen Methoden, vor oder während der Formgebung, oder auch durch Aufbringen der gelösten oder dispergierten Verbindungen auf das Polymere

direkt oder Einmischen in eine Lösung, Suspension oder Emulsion desselben, gegebenenfalls unter nachträglichem Verdunstenlassen des Lösungsmittels, erforlgen. Die Mengen liegen bei 0,01 bis 5, vorzugsweise bei 0,05 bis 2,5 und insbesondere bei 0,1 bis 1,0 Gew.-%, bezogen auf das zu stabilisierende Material. Die neuen Verbindungen können auch in form eines Masterbatches, der diese Verbindungen beispielsweise in einer Konzentration von 1 bis 50, vorzugsweise 2,5 bis 20 Gew.-% enthält, den zu stabilisierenden Kunststoffen zugesetzt werden.

Die durch den Zusatz der erfindungsgemäßen Substanzen stabilisierten Kunststoffe können gegebenenfalls noch andere bekannte und übliche Zusätze, wie beispielsweise Antioxidantien auf Phenol- und Sulfidbasis, Metalldesaktivatoren und Lichtschutzmittel, Phosphitstabilisatoren, Metallverbindungen, Epoxistabilisatoren und mehrwertige Alkohole enthalten (s.a. DE—OS 24 27 853, S. 18—24).

Beispiele für Antioxidantien sind sterisch gehinderte Phenole wie 2,6-Di-tert.-butyl-4-methylphenol, 4,4'-Butyliden-bis-(2,6-di-tert.-butylphenol), 4,4'-Thio-bis-(2-tert.-butyl-5-methylphenol), 2,5-Di-tert.-butyl-4-hydroxianisol, 2,2-Bis-(3,5-di-tert.-butyl-2-hydroxibenzyl)-malonsäuredioctadecylester, 1,3,5-Tris-(3,5-di-tert.-butyl-4-hydroxibenzyl)-2,4,6-trimethylbenzol, 2,4,6-Tri-(3,5-di-tert.-butyl-4-hydroxibenzyl)-phenol, phenolische Triazinverbindungen wie 1,3,5-Tris-(3,5-di-tert.-butyl-4-hydroxibenzyl)isocyanurat, Ester der β-(3,5-Di-tert.-butyl-4-hydroxiphenyl)-propionsäure mit z.B. Octadecanol, Pentaerythrit und Tris-hydroxiethyl-isocyanurat, Ester der 3,3-Bis-(3-tert.-butyl-4-hydroxiphenyl)-butansäuren mit z.B. Ethylenglykol, Thiodipropionsäureestern mit Fettalkoholen, Ca- oder Ni-Salze des 3,5-Di-tert.-butyl-4-hydroxibenzylphosphonsäureethylesters, Dioctadecylsulfid und -disulfid.

Zu den UV-Absorbern und Lichtschutzmitteln gehören 2-(2'-Hydroxiphenyl)-benztriazole wie z.B. das 5-Chlor-3',5'-di-tert.-butyl und 5-Chlor-3',5'-di-tert.-amyl-Derivat, 2-Hydroxibenzophenone wie z.B. das 4-Heptoxi- oder 4-Octoxi-Derivat, Salizylate wie Octylphenylsalizylat, Nickelkomplexe wie z.B. die das 2,2'-Thio-bis-4-(1,1,3,3-tetramethylbutyl)-phenols mit Butylamin oder anderen Aminen, Oxalsäurediamide und sterisch gehinderte Amine.

Als Phosphite sind aliphatische, aromatische oder aliphatischaromatische wie z.B. Trisnonylphenylphosphit, Tris-(2,4-di-tert.-butylphenyl)-phosphit, Tris-(2-tert.-butylphenyl)-phosphit oder auch Ester des Pentaerythritphosphits zu nennen.

Unter als Stabilisatoren bekannten Metallverbindungen werden in diesem Zusammenhang verstanden: Calcium-, Barium-, Strontium-, Zink-, Cadmium-, Magnesium-, Aluminium- und Bleiseifen aliphatischer Carbonsäuren oder Oxicarbonsäuren mit etwa 12 bis 32 C-Atomen, Salze der genannten Metalle mit aromatischen Carbonsäuren wie Benzoate oder Salizylate sowie (Alkyl)-Phenolate dieser Metalle, ferner Organozinnverbindungen, wie z.B. Dialkylzinnthioglykolate und -carboxilate.

Bekannte Epoxistabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 2 bis 6 OH-Gruppen.

Eine wirksame Stabilisatorkombination für Poly-α-Olefine, wie z.B. Hoch-, Mittel- und Niederdruckpolymerisate von $C_2$- bis $C_4$-α-Olefinen, insbesondere Polyethylen und Polypropylen oder von Copolymerisaten derartiger α-Olefine besteht, bezogen auf 100 Gew.-Teile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat oder der entsprechenden Oxide sowie gegebenenfalls 0,01 bis 5 Gewichtsteilen eines bekannten UV-Stabilisators aus der Gruppe der Alkoxihydroxibenzophenone, 4-Hydroxiphenylbenzotriazole, Benzylidenmalonsäuremononitrilester oder der sog. Quencher, wie z.B. Nickelchelate. Als sonstige übliche Zusätze sind z.B. Weichmacher, Gleitmittel, Emulgatoren, Füllstoffe wie z.B. Kreide, Talkum, Asbest, Pigmente, optische Aufheller, Flammschutzmittel und Antistatika anzusehen.

Die erfindungsgemäß stabilisierten Kunststoffe können in verschiedenster Form angewendet werden, z.B. als Folien, Fasern, Bändchen, Profile oder als Bindemittel für Lacke, Klebemittel oder Kitte.

Der weiteren Erläuterung der Erfindung dienen nachstehende Beispiele. Hierbei zeigen die Beispiele 1 bis 13 die Herstellung von Verbindungen der Formel (III), welche dann nach den Beispielen 14 bis 47 mit einem Diamin der Formel (IV) zu den erfindungsgemäßen Poly-bis-triazinylaminen umgesetzt werden. Sowohl die Zwischenprodukte als auch die Oligomeren besitzen keinen scharfen Schmelzpunkt, sondern einen Schmelzbereich.

## Beispiel 1

N,N'-Bis-[2-Chlor-4-<(2,2,6,6-tetramethyl-4-piperidyl)-3-methoxipropylamino>-1,3,5-triazin-6-yl]-N-(2,2,6,6-tetramethyl-4-piperidyl)-1,6-diaminohexan

In einer 1-l-Rührapparatur werden 260 g Toluol und 120 g Aceton vorgelegt. Man fügt 36,8 g (0,2 Mol) Cyanurchlorid zu und kühlt die Lösung auf 0°C ab. Dann tropft man bei 0 bis 10°C Innentemperatur 45,6 g (0,02 Mol) N-(2,2,6,6-Tetramethyl-4-piperidyl)-3-methoxipropylamin (= Verbindung der Formel (VI)) zu und anschließend bei 5 bis 10°C innerhalb von 3 Stunden 32 g 25%ige wäßrige Natronlauge. Nach Zugabe von 25,5 g (0,1 Mol) N-(2,2,6,6-Tetramethyl-4-piperidyl)-1,6-diaminohexan (= Verbindung der Formel (VIII)) wird auf 50°C erwärmt und bei dieser Temperatur innerhalb von 2 Stunden 32 g 25%ige wäßige Natronlauge tropfenweise zugesetzt. Nach Zugabe von 80 ml Aceton und 60 ml Wasser wird die org. Phase abgetrennt,

bei ca. 70°C Heizbadtemperatur im Vakuum zur Trockene eingeengt und bei 70°C im Hochvakuum getrocknet. Es bleiben 91,5 g = 98% d. Th. eines farblosen Feststoffes vom Fp. ca. 100°C (zähe Schmelze) zurück.

Beispiele 2 bis 13

Nach den Angaben des Beispiels 1 werden weitere Verbindungen (III) aus Cyanurchlorid und den in Tabelle 1 aufgeführten Komponenten hergestellt. Die in Spalte 4 der Tabelle eingetragenen Nummern verweisen auf die Aufstellung der Verbindungen auf den Seiten 10 und 11.

TABELLE 1

| Bsp. Nr. | Ausgangsverbindungen | | Erh. Prod. d. Formel (III) | | |
|---|---|---|---|---|---|
| | monofunktionelle Komponente (VI) g ≙ Mol | Diamin (VIII) g ≙ Mol | Verb. Nr. | Ausbeute g ≙ % d.Th. | Fp (°C) |
| 2 | analog Beispiel 1 | N-(2,2,6,6-Tetramethyl-4-piperidyl)-1,3-bis-(amino-methyl)-cyclohexan 28,1 ≙ 0,1 | 2 | 95 ≙ 99 | 120 |
| 3 | dto. | N-(2,2,6,6-Tetramethyl-4-piperidyl)-3-(4),8(9)-bis-amino-methyl)-tricyclo-[5.2.1.$0^{2,6}$]-decan-Isomeren-gemisch 33,3 ≙ 0,1 | 3 | 100 ≙ 99 | 100 |
| 4 | N-(2,2,6,6-Tetramethyl-4-piperidyl)-3-ethoxi-propylamin 48,4 ≙ 0,2 | N,N'-Bis-(2,2,6,6-tetramethyl-4-piperidyl)-4,9-dioxadodecan-1,12-diamin 48,2 ≙ 0,1 | 4 | 118 ≙ 99 | 60 |
| 5 | N-(2,2,6,6-Tetramethyl-4-piperidyl)-butylamin 42,5 ≙ 0,2 | N,N'-Bis-(2,2,6,6-tetramethyl-4-piperidyl)-1,6-diaminohexan 39,4 ≙ 0,1 | 5 | 104 ≙ 100 | 90 |
| 6 | dto. | N,N'-Bis-(cyclododecyl)-1,6-diaminohexan 45,0 ≙ 0,1 | 6 | 109 ≙ 99 | 95 |
| 7 | N-(2,2,6,6-Tetramethyl-4-piperidyl)-butylamin 42,5 ≙ 0,2 | N-Cyclohexyl-1,3-diamino-propan 15,6 ≙ 0,1 | 7 | 80 ≙ 99 | 105 |
| 8 | Dibutylamin 25,8 ≙ 0,2 | N,N'-Bis-(2,2,6,6-tetramethyl-4-piperidyl)-ethylendiamin 33,8 ≙ 0,1 | 8 | 81 ≙ 99 | Harz |

Fortsetzung TABELLE 1

| Bsp. Nr. | Ausgangsverbindungen | | Erh. Prod. d. Formel (III) | | |
|---|---|---|---|---|---|
| | monofunktionelle Komponente (VI) g ≙ Mol | Diamin (VIII) g ≙ Mol | Verb. Nr. | Ausbeute g ≙ % d.Th. | Fp (°C) |
| 9 | Morpholin<br>17,4 ≙ 0,2 | dto. | 9 | 49 ≙ 67 | Harz |
| 10 | Octadecylamin<br>53,8 ≙ 0,2 | analog Beispiel 8 | 10 | 106 ≙ 69 | 145 |
| 11 | N-(2,2,6,6-Tetramethyl-4-piperidyl)-octadecylamin<br>81,6 ≙ 0,2 | Piperazin<br>8,6 ≙ 0,1 | 11 | 78 ≙ 69 | 80 |
| 12 | analog Beispiel 4 | Bis-(3-aminopropyl)-piperazin<br>20 ≙ 0,1 | 12 | 75 ≙ 83 | Harz |
| 13 | Phenol<br>18,8 ≙ 0,2 | analog Beispiel 8 | 13 | 69 ≙ 80 | 85 |

Beispiel 14

Poly-bis-triazinylamin aus Verbindung 1 und Hexamethylendiamin

In einer 250-ml-Rührapparatur werden 18,0 g (0,019 Mol) der nach Beispiel 1 erhaltenen Verbindung zusammen mit 2,3 g (0,02 Mol) Hexamethylendiamin und 1,6 g (0,04 Mol) NaOH-Pulver in 100 ml Xylol 9 Stunden unter Rückfluß (132°C) gerührt. Anschließend wird filtriert, das Filtrat im Vakuum einrotiert und der Rückstand 4 Stunden am Hochvakuum bei 150°C getrocknet. Es bleibt ein nahezu farbloses Harz vom Fp. 115°C in quantitativer Ausbeute zurück. Molgewicht 3300.

Beispiele 15 bis 47

Man arbeitet analog Beispiel 14 unter Einsatz weiterer Verbindungen der Formel (III). In Tabelle 2 sind die Versuchsdaten zusammengestellt. Spalte 2 verweist auf die in Tabelle 1, Spalte 4, eingetragene Verbindungsnummer, die sich auf die Aufstellung auf den Seiten 10 und 11 bezieht.

TABELLE 2

| Bsp. Nr. | Ausgangsmaterialien | | Endprodukt |
|---|---|---|---|
| | Bis-(2-chlor-4-aminotriazinyl)-diamin (III) g ≙ Mol | Diamin (IV) g ≙ Mol | Fp (τ) des Polymerisats |
| 15 | analog Beispiel 14 | 1,3-Bis-(aminomethyl)-cyclohexan 2,85 ≙ 0,02 | 150 |
| 16 | dto. | 4,7-Dioxadecan-1,10-diamin 2,90 ≙ 0,02 | 110 |
| 17 | dto. | N,N'-Bis-(2,2,6,6-tetramethyl-4-piperidyl)-1,6-diaminohexan 7,9 ≙ 0,02 | 115 |
| 18 | dto. | 3(4),8(9)-Bis-(aminomethyl)-tricyclo-[5.2.1.$0^{2,6}$]-decan-Isomeren-gemisch 3,85 ≙ 0,02 | 165 |
| 19 | Verbindung Nr. 2 19,2 ≙ 0,02 | Ethylendiamin 1,2 ≙ 0,02 | 155 |
| 20 | dto. | 4,4'-Diaminodicyclohexyl-methan 4,1 ≙ 0,02 | 155 |
| 21 | dto. | 7-Methyl-4,10-dioxa-tridecan-1;13-diamin 4,65 ≙ 0,02 | 130 |
| 22 | dto. | N-(2,2,6,6-Tetramethyl-4-piperidyl)-1,6-diaminohexan, 5,1 ≙ 0,02 | 125 |
| 23 | Verbindung Nr. 2 19.2 ≙ 0,02 | Bis-(3-aminopropyl)-piperazin 4,0 ≙ 0,02 | 150 |
| 24 | Verbindung Nr. 3 23,0 ≙ 0,02 | 1,3-Diaminopropan 1,5 ≙ 0,02 | 140 |
| 25 | dto. | analog Beispeil 18 | 130 |
| 26 | dto. | 1,6-Diaminohexan 2,3 ≙ 0,02 | 130 |
| 27 | dto. | N-(2,2,6,6-Tetramethyl-4-piperidyl)-ethylendiamin 4,0 ≙ 0,02 | 110 |
| 28 | dto. | 3-Amino-1-methylaminopropan 1,8 ≙ 0,02 | 110 |
| 29 | Verbindung Nr. 5 20.8 ≙ 0.02 | analog Beispiel 26 | 85 |
| 30 | dto. | Ethylendiamin 1,2 ≙ 0,02 | 80 |
| 31 | dto. | analog Beispiel 18 | 80 |

Fortsetzung TABELLE 2

| Bsp. Nr. | Ausgangsmaterialien | | Endprodukt |
|---|---|---|---|
| | Bis-(2-chlor-4-aminotriazinyl)-diamin (III) g ≙ Mol | Diamin (IV) g ≙ Mol | Fp (τ) des Polymerisats |
| 32+) | Verbindung Nr. 6<br>21,0 ≙ 0,02 | analog Beispiel 17 | 160 |
| 33 | dto. | analog Beispiel 27 | 180 |
| 34+) | dto. | N,N'-(2,2,6,6-Tetramethyl-4-piperidyl)-ethylendiamin<br>6,9 ≙ 0, 02 | 175 |
| 35+) | dto. | N-(2,2,6,6-tetramethyl-4-piperidyl)-3(4),8(9)-bis-(aminomethyl)-tricyclo-[5.2.1.$0^{2,6}$]-decan-Isomerengemisch<br>9,4 ≙ 0,02 | 160 |
| 36 | Verbindung Nr. 7<br>16,05 ≙ 0,02 | analog Beispiel 27 | 240 |
| 37 | dto. | analog Beispiel 18 | 200 |
| 38 | dto. | analog Beispiel 35 | 160 |
| 39 | Verbindung Nr. 4<br>23,7 ≙ 0,02 | analog Beispiel 14 | 130 |
| 40 | Verbindung 4<br>23,7 ≙ 0,02 | 4,9-Dioxadodecandiamin-1,12<br>4,1 ≙ 0,002 | 120 |
| 41 | dto. | p-Phenylendiamin<br>2,3 ≙ 0,02 | 80 |
| 42 | analog Beispiel 29 | 1,8-Diamino-p-menthan<br>3,4 g ≙ 0,02 | 140 |
| 43 | Verbindung Nr. 11<br>22,5 g ≙ 0,02 | analog Beispiel 34 | 120 |
| 44 | Verbindung Nr. 12<br>18,1 g ≙ 0,02 | analog Beispiel 27 | 145 |
| 45 | Verbindung Nr. 9<br>14,7 ≙ 0,02 | analog Beispiel 19 | 200 |
| 46 | Verbindung Nr. 8<br>16,4 ≙ 0,02 | dto. | 150 |
| 47 | Verbindung Nr. 13<br>15,0 ≙ 0,02 | analog Beispiel 35 | 170 |

+) Lösungmittel Mesitylen

Beispiel 48

In einer 250-ml-Rührapparatur werden in 40 ml Xylol 12,05 g (1/20 Mol) N-p(2,2,6,6,-Tetramethyl-4-piperidyl)-3-dimethylaminopropylamin vorgelegt. Sodann werden bei −5 bis + 5°C Innentemperatur 9,2 g (1/20 Mol) Cyanurchlorid gelöst in 60 ml Xylol zugetropft. Man erwärmt auf 10°C, fügt 2,9 g (1/20 Mol) NaOH-Pulver zu und rührt 3 Stunden bei 10°C. Es werden sodann 5,0 g (1/40 Mol) N-(2,2,6,6-Tetramethyl-4-piperidyl)-ethylendiamin zugegeben, worauf man auf 50°C erwärmt und nach Zugabe von 2,0 g (1/20 Mol) NaOH-Pulver 5 Stunden bei dieser Temperatur rührt. Nach Zugabe von 1,5 g (1/40 Mol) Ethylendiamin und 2,0 g (1/20 Mol) NaOH-Pulver wird nochmals 8 Stunden unter Rückfluß gerührt, dann filtriert und das Filtrat im Vakuum zur Trockene eingeengt. Der Rückstand wird bei 150°C im Hochvakuum getrocknet. Es bleibt ein blaßgelbes festes Harz in nahezu quantitativer Ausbeute. Fp 140°C.

Beispiel 49

In einer 250-ml-Rührapparatur werden in 40 ml Xylol, 9,2 g (1/20 Mol) Cyanurchlorid vorgelegt. Hierzu tropft man bei 0 bis 10°C 12,0 g (1/20 Mol) N-(2,2,6,6,-Tetramethyl-4-piperidyl)-aminohexan gelöst in 60 ml Xylol. Nach Zugabe von 2,0 g (1/20 Mol) NaOH-Puylver wird 3 Stunden bei 10°C gerührt und dann wie im Beispiel 40 angegeben weiter verfahren, wobei ein farbloses Harz anfällt. Fp. 215°C.

Beispiel 50 bis 52

Es wird wie in Beispiel 49 gearbeitet unter Einsatz nach stehender Substanzen:

| Bsp Nr. | Ausgangsstoffe | | | Verfahrensprod. Fp (°C) |
|---|---|---|---|---|
| | Monoamin (VI) g ≙ Mol | Diamin (VIII) g ≙ Mol | Diamin (IV) g ≙ Mol | |
| 50 | Piperidin 4,25 ≙ 1/20 | anal. Bsp. 35 | anal. Bsp. 18 | 230 |
| 51 | Dioctadecyl-amin 22,3 ≙ 1/10 | anal. Bsp. 17 | anal. Bsp. 24 | 85 |
| 52 | Diethanol-amin 5,25 ≙ 1/20 | anal. Bsp. 22 | anal. Bsp 27 | 155 |

Beispiel 53

Analog Beispiel 1 wird die Verbindung 14 aus 45,2 g (0,2 Mol) 2-Phenyl-4,6-dichlor-1,3,5-triazin und 48,2 g (0,1 Mol) des nach Beispiel 4 erhaltenen Diamins erhalten. Ausbeute 67 g ≙ 78%, Fp. 60°C.

Beispiel 54

Dieses Beispiel zeigt die Flüchtigkeit der neuen Triazinstabilisatoren im Vergleich zu einem Produkt des nächsten Standes der Technik.

Die Flüchtigkeiten wurden in einer Apparatur zur thermogravimetrischen Analyse bestimmt. Gleiche Menge (500 mg) der erfindungsgemäßen Verbindungen und der Vergleichyssubstanz wurden dazu in Stickstoffatomosphäre mit einer Aufheizgeschwindigkeit von 2 K/min. bis auf 300°C erhitzt und der Substanzverlust in mg/cm$^2$ Probenoberfläche gemessen. Die Ergebnisse zeigt nachstehende Tabelle.

| Stabilisator gem. Beispiel | Gewichtsverlust in mg/cm² beim Erreichen von . . .°C | | | |
|---|---|---|---|---|
| | 220 | 260 | 300 | 10 Min. bei 300°C |
| 25 | 0,31 | 2,37 | 6,00 | 7,58 |
| 14 | 0,16 | 2,05 | 4,42 | 5,21 |
| 18 | 0,31 | 0,95 | 2,84 | 3,36 |
| 26 | 0,16 | 1,58 | 3,48 | 4,42 |
| Vergleich +) | 0,47 | 3,48 | 10,59 | 17,38 |

+) Substanz nach Beispiel 6 der DE—OS 26 36 144

Beispiel 55

Dieses Beispiel soll die lichtstabilisierende Wirkung der neuen Verbindungen in Poly-α-Olefinen aufzeigen.

100 Gewichtsteile Polypropylen mit dem Schmelzindex $i_5$ (230°C) von ca. 6 g/10 Min. (bestimmt nach ASTM D 1238—62 T) und einer Dichte von 0,90 wurden mit

0,1 Gew.-T. Pentaerythrityl-tetrakis-3-(3,5-di-tert.-butyl-4-hydroxiphenyl)-propionat,

0,2 Gew.-T Calciumstearat und

0,1 Gew.-T. des zu prüfenden erfindungsgemäßen Stabilisators vermischt.

Um eine möglichst gleichmäßige Verteilung auf dem Polymerkorn zu erreichen, wurden die Stabilisatoren in einem Lösemittel gelöst, und die Lösung wurde unter Rühren in das Polypropylenpulver eingetropft, wobei durch gleichzeitige Bestrahlung mit einer IR-Lampe der größte Teil des Lösemittels wieder abdampfte.

Nach ca. 20 Minuten wurde das Calciumstearat hinzugegeben und noch weitere 10 Minuten gemischt. Lösemittelreste wurden durch Trocknen bei 50°C/120 Min. im Trockenschrank entfernt.

Das Polypropylen wurde auf einer Windsor-Spritzgußmaschine der Type SP 50 bei 240°C zu 60 × 60 × 1 mm-Platten verspritzt. Aus diesen Platten wurden Prüfkörper nach DIN 53 455, Form 3, verkleinert im Maßstab 1:3, ausgestanzt. Die als Vergleichsmuster benötigten Prüfkörper wurden analog. jedoch unter Fortlassen des zu testenden Stabilisators bzw. unter Zusatz der Vergleichsstabilisatoren, hergestellt.

Zur Bestimmung der Lichtstabilität wurden die Proben in einer Xenotest-1200-Apparatur der Firma Original Hanau Quarzlampen GmbH der Bestrahlung mit Wechsellicht unterworfen. Die Strahlungsintensität wurde durch UV-Filter (Spezialfilterglas d = 1,7 mm) moduliert. die Lichtbeständigkeit wurde nach DIN 53 387 (17 Min. Trockenperiode, 3 Min. beregnen, Schwarztafeltemperatur 45°C, relative Luftfeuchtigkeit während der Trockenperiode 70 bis 75%) geprüft. Gemessen wurde die Reißdehnung auf einer Zugprüfmaschine der Firma Instron bei einer Abzugsgeschwindigkeit von 5 cm/Min. nach einer bestimmten Belichtungszeit in Stunden.

| Stabilisator nach Bsp. | Belichtungszeit in Stunden | ermittelte Reißdehnung in % vom Ausgangswert |
|---|---|---|
| 14 | 1 400 | >50 |
| 25 | 1 400 | >50 |
| 26 | 1 400 | >50 |
| Polypropylen | 260 | 1 |
| Vergleich 1) | 320 | 1 |
| Vergleich 2) | 1 400 | 50 |

1) ohne Lichtstabilisator

2) Verbindung nach Beispiel 6 der DE—OS 26 36 144

Beispeil 56

In einem Laborschnellmischer wird aus Polypropylenpulver (® Hostalen PPU VP 1770 F der Hoechst AG) vom Schmelzindex MFI 190/5 = 1,9 g/10 Min. s. DIN 53 535 und den unten angegebenen Rezepturbestandteilen eine homogene Mischung hergestellt und in ein Granulat überführt. Das so stabilisierte Material wird sodann in einem Laborextruder unter den üblichen Verarbeitungsbedingungen aufgeschmolzen und über eine Spinnpumpe mit Achtfachspinnkopf zu Monofilamenten verarbeitet, welche anschließend im Verhältnis 1:3 nachverstreckt, zu Garn von 40 dtex texturiert und zu Prüfgeweben verarbeitet werden.

100 Gew.-T. Polypropylen,
0,2 Gew.-T. Calciumstearat,
0,1 Gew.-T. 3,3-Bis-(3-tert.-butyl-4-hydroxiphenyl)-butansäureethylenglykolester,
0,1 Gew.-T. Dioctadecyldisulfid,
0,3 Gew.-T. des zu prüfenden Stabilisators

Die Gewebeproben werden auf einen gelochten Karton so aufgespannt, daß eine freie Öffnung von ca. 15,5 mm Durchmesser bestehen bleibt. In dieser Form werden die Prüflinge im Xenotest X 1200 nach dem vorhergehenden Beispiel belichtet. In bestimmten Zeitabständen werden die Gewebe zentrisch mit einem Gewicht von 6 mm Durchmesser und einem Druck von 0,1 N/mm² belastet. Als Versagenstermin gilt das Durchbrechen des Gewichts.

| Stabilisator nach Beispiel | Belichtungszeit in Stunden |
|---|---|
| 25 | 3 100 [4] |
| 14 | 3 100 [4] |
| 26 | 3 100 [4] |
| Polypropylen | < 280 |
| Vergleich [1] | 400 |
| Vergleich [2] | 1 400 |
| Vergleich [3] | 3 000 |

[1] ohne Stabilisator
[2] Verbindung nach Bsp. 1 der DE—OS 27 19 131 (Handelsprodukt Tinuvin 622)
[3] Verbindung nach Bsp. 6 der DE—OS 26 36 144
[4] Gewicht noch nicht durchgebebrochen

Beispiel 57

Das wie im vorhergehenden Beispiel hergestellte stabilisierte Granulat wird auf einer Labor-Folienblasanlage (Schneckendurchmesser 25 mm, Länge 20 D), Temperaturprogramm 200, 240, 250, 255°C) zu Blasfolien von ca. 70 µ Dichtevberarbeitet. Diese Folien werden im Xenotest X 1200 wie beschrieben künstlich bewittert. Als Schädigungsmerkmal wird die Carbonylzahl in Anlehnung an DIN 53 383, Teil 2, gemessen. (Diese wird für PP definiert als das Verhältnis der Extinktionen bei 1715 $cm^{-1}$ und 1524 $cm^{-1}$. Bei einer CO-Zahl >2 beginnt der Zerfall der Prüfkörper zu Pulver.)

| Stabilisator nach Beispiel | C=O-Zahl nach ... Stunden | | | |
|---|---|---|---|---|
| | 500 | 1000 | 2000 | 2500 |
| 25 | <0,1 | <0,1 | 0,3 | 0,4 |
| 14 | <0,1 | <0,1 | 0,3 | 0,5 |
| 26 | <0,1 | <0,1 | 0,3 | 0,5 |
| Polypropylen | >2 | — | — | — |
| Vergleich [1] | >2 | — | — | — |
| Vergleich [2] | — | >2 | — | — |
| Vergleich [3] | <0,1 | <0,1 | 0,4 | 0,9 |

[1], [2] und [3] entsprechen den Vergleichsproben des vorigen Beispiels

**Patentansprüche für die Vertragsstaaten: BE CH DE FR GB IT LI NL SE**

1. Poly-bis-triazinylamine der allgemeinen Formel (I)

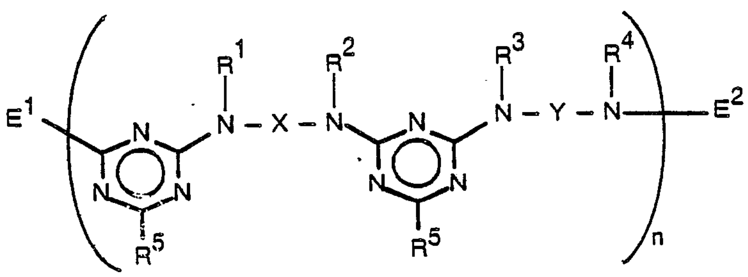

(I)

in der

n eine ganze Zahl von 1 bis 200 ist,

$R^1$ und $R^2$ gleich oder verschieden sind und Wasserstoff, $C_1$- bis $C_{37}$-Alkyl, unsubstituiertes oder durch bis zu vier $C_1$- bis $C_4$-Alkylgruppen substituiertes Cycloalkyl, $C_7$- bis $C_{14}$-Aralkyl oder eine Gruppe der Formel (II),

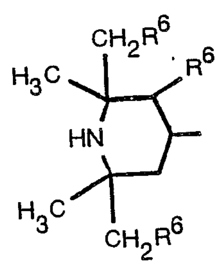

(II),

in welcher $R^6$ Wasserstoff oder $C_1$- bis $C_6$-Alkyl darstellt, bedeuten,

$R^3$ und $R^4$ gleich oder verschieden sind und die unter $R^1$ und $R^2$ angegebenen Bedeutungen haben,

$R^5$ Halogen, Phenyl, $—OR^7$ oder $—NR^8R^9$ ist, wobei $R^7$ für Wasserstoff, $C_1$- bis $C_{18}$-Alkyl, $C_3$- bis $C_{12}$-Alkenyl, $C_5$- bis $C_{12}$-Cycloalkyl, $C_7$- bis $C_{18}$-Aralkyl, $C_6$- bis $C_{10}$-Aryl oder einen Rest der Formel (II) steht und

$R^8$ und $R^9$ gleich oder verschieden sind und Wasserstoff, unsubstituiertes oder durch eine Hydroxi-, $C_1$- bis $C_{18}$-Alkoxi- oder $C_1$- bis $C_4$-Dialkylamino gruppe substituiertes $C_1$- bis $C_{37}$-Alkyl, $C_3$- bis $C_{12}$-Alkenyl, $C_5$- bis $C_{12}$-Cycloalkyl, $C_7$- his $C_{18}$-Aralkyl, Phenyl, Naphthyl oder den Rest der Formel (II) bedeuten, oder auch

$R^8$ und $R^9$ zusammen mit dem gemeinsamen Stickstoffatom einen Pyrrolidin- oder einen unsubstituierten oder durch bis zu vier $C_1$- bis $C_4$-Alkylgruppen substituierten Piperidin-, Morpholin- oder Hexamethyleniminring bilden,

X und Y gleich oder verschieden sind und für $C_2$- bis $C_{18}$-Alkylen oder für $C_2$- bis $C_{12}$-Bis-(propyloxi)-alkylen oder für unsubstituiertes oder durch bis zu vier Methylgruppen substituiertes $C_6$- bis $C_{18}$-Mono-, Di- oder Tricycloalkylen, in welchem bei dem erstgenannten zwei C-Atome durch N-Atome, welche Propylengruppen tragen können, ersetzt sein können, oder für $C_6$- bis $C_{18}$-Arylen oder für $C_7$- bis $C_{18}$-Aralkylen stehen, oder auch anstelle der Gruppierungen

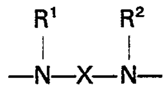

oder

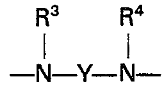

Piperazinylen-Reste treten,

$E^1$ ein Halogenatom oder eine Gruppe

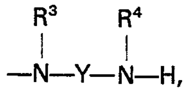

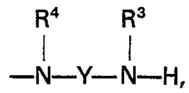

$-O-R^7$, oder $-NR^8R^9$ und

$E^2$ Wasserstoff oder eine Acylgruppe ist, wobei $R^3$, $R^4$, $R^7$, $R^8$ und $R^9$ die oben angegebenen Bedeutungen haben, und mindestens einer der Reste $R^1$, $R^2$ $R^3$, $R^4$, $R^7$, $R^8$ und $R^9$ eine Gruppe der Formel (II) sein muß, mit der Bedingung, daß $R^1$ bis $R^4$ gleich sind, wenn X und Y verschieden sind, und X und Y gleich sind, wenn mindestens einer der Reste $R^1$ oder $R^2$ von $R^3$ und $R^4$ verschieden ist.

2. Verfahren zur Herstellung von Poly-bis-triazinylaminen nach Anspruch 1, dadurch gekennzeichnet, daß man in einem inerten, organischen Lösungsmittel entweder

A. ein Dihalogen-bis-triazinyldiamin der Formel (III)

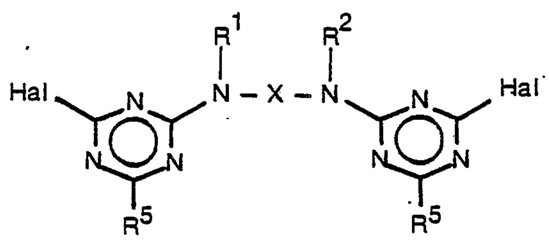

(III)

mit der 0,8- bis 1,4 fach molaren Menge eines Diamins der Formel (IV)

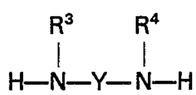

(IV),

wobei in diesen Formeln Hal, $R^1$, $R^2$ $R^3$, $R^4$, $R^5$. X und Y die in Anspruch 1 angegebenen Bedeutungen haben, in Gegenwart der—bezogen auf Verbindung (IV)—äquivalenten Menge einer als Halogenwasserstoffakzeptor dienenden anorganischen Base bei 50 bis 200°C umsetzt, oder

B. ein Poly-bis-triazinylamin der allgemeinen Formel (V)

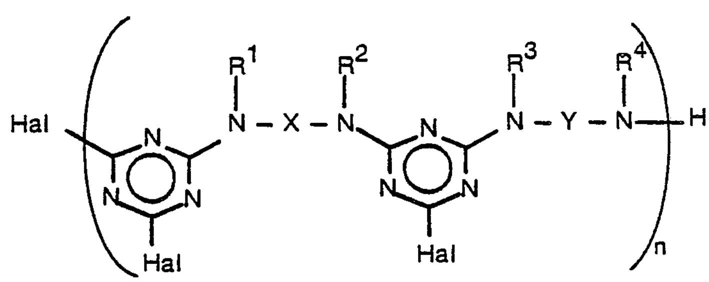

(V)

mit der 0,8- bis 1,4 fach valenten Menge einer Verbindung der Formel (VI)

$$H-R^5$$ (VI),

wobei in diesen Formeln Hal, $R^1$, $R^2$ $R^3$, $R^4$, $R^5$. X und Y die in Anspruch 1 angegebenen Bedeutungen haben, jedoch $R^5$ nicht Hal oder Phenyl ist, in Gegenwart der—bezogen auf Verbindung (VI)—äquivalenten Menge einer anorganischen Base bei 50 bis 200°C zur Reaktion bringt, oder

C. zunächst bei −5 bis 40°C in Gegenwart der äquivalenten Menge eines Halogenwasserstoffakzeptors, Cyanurhalogenid und die 0,8- bis 1,3 fach molare Menge einer Verbindung der Formel (VI), wobei jedoch $R^5$ nicht Hal oder Phenyl ist, zu einem Dihalogentriazin der Formel (VII)

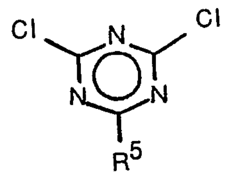

(VII),

umsetzt, dieses anschließend ohne Zwischenisolierung bei 10 bis 70°C in Gegenwart der äquivalenten Menge eines Halogenwasserstoffakzeptors durch Reaktion mit der 0,4- bis 0,7 fach molaren Menge eines Diamins der Formel (VIII)

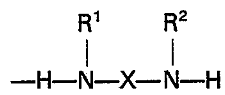

(VIII),

zu einem Dihalogen-bis-triazinyldiamin der Formel (III) derivatisiert, und die so erhaltenen Verbindungen (III) is situ gemäß Verfahren A zu den gewünschten Endprodukten umsetzt, wobei in den Formeln Hal, $R^1$, $R^2$ $R^3$, $R^4$, $R^5$. X und Y die in Anspruch 1 angegebenen Bedeutungen haben.

3. Verwendung der Verbindungen nach Anspruch 1 zum Stabilisieren von synthetischen Polymeren.

4. Verwendung nach Anspruch 3, dadurch gekennzeichnet, daß das Polymere ein Polyolefin ist.

5. Verwendung nach Anspruch 3, dadurch gekennzeichnet, daß das Polymere ein Polyacrylat oder Polymethacrylat ist.

6. Verwendung nach Anspruch 3, dadurch gekennzeichnet, daß das Polymere ein Homo- oder Copolymerisat des Styrols ist.

7. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

8. Gegen UV-Zersetzung stabilisierte, synthetische Polymere, in denen 0,01 bis 5 Gewichtseile, bezogen auf Polymeres, eines Stabilisators nach Anspruch 1 enthalten sind.

**Patentansprüche für den Vertragsstaat: AT**

1. Verfahren zur Herstellung von Poly-bis-triazinylaminen der allgemeinen Formel (I)

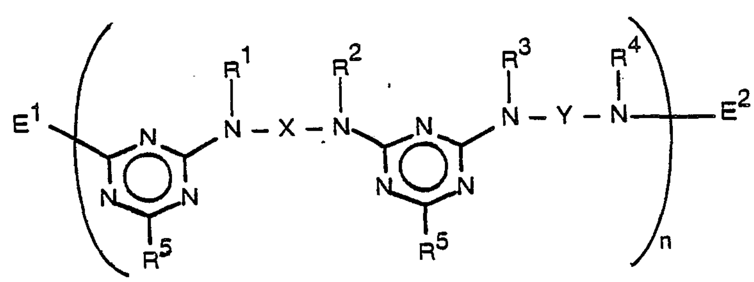

(I)

in der

n eine ganze Zahl von 1 bis 200 ist,

$R^1$ und $R^2$ gleich oder verschieden sind und Wasserstoff, $C_1$- bis $C_{37}$-Alkyl, unsubstituiertes oder durch bis zu vier $C_1$- bis $C_4$-Alkylgruppen substituiertes Cycloalkyl, $C_7$- bis $C_{14}$-Aralkyl oder eine Gruppe der Formel (II),

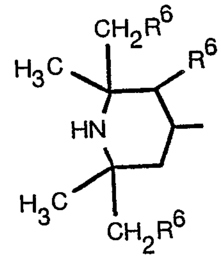 (II),

in welcher $R^6$ Wasserstoff oder $C_1$- bis $C_6$-Alkyl darstellt, bedeuten,

$R^3$ und $R^4$ gleich oder verschieden sind und die unter $R^1$ und $R^2$ angegebenen Bedeutungen haben,

$R^5$ Halogen, Phenyl, —$OR^7$ oder —$NR^8R^9$ ist, wobei $R^7$ für Wasserstoff, $C_1$- bis $C_{18}$-Alkyl, $C_3$- bis $C_{12}$-Alkenyl, $C_5$- bis $C_{12}$-Cycloalkyl, $C_7$- bis $C_{18}$-Aralkyl, $C_6$- bis $C_{10}$-Aryl oder einen Rest der Formel (II) steht und

$R^8$ und $R^9$ gleich oder verschieden sind und Wasserstoff, unsubstituiertes oder durch eine Hydroxi-, $C_1$- bis $C_{18}$-Alkoxi- oder $C_1$- bis $C_4$-Dialkylamino gruppe substituiertes $C_1$- bis $C_{37}$-Alkyl, $C_3$- bis $C_{12}$-Alkenyl, $C_5$- bis $C_{12}$-Cycloalkyl, $C_7$- bis $C_{18}$-Aralkyl, Phenyl, Naphthyl oder den Rest der Formel (II) bedeuten, oder auch

$R^8$ und $R^9$ zusammen mit dem gemeinsamen Stickstoffatom einen Pyrrolidin- oder einen unsubstituierten oder durch bis zu vier $C_1$- bis $C_4$-Alkylgruppen substituierten Piperidin-, Morpholin- oder Hexamethyleniminring bilden,

X und Y gleich oder verschieden sind und für $C_2$- bis $C_{18}$-Alkylen oder für $C_2$- bis $C_{12}$-Bis-(propyloxi)-alkylen oder für unsubstituiertes oder durch bis zu vier Methylgruppen substituiertes $C_6$- bis $C_{18}$-Mono-, Di- oder Tricycloalkylen, in welchem bei dem erstgenannten zwei C-Atome durch N-Atome, welche Propylengruppen tragen können, ersetzt sein können, oder für $C_6$- bis $C_{18}$-Arylen oder für $C_7$- bis $C_{18}$-Aralkylen stehen, oder auch anstelle der Gruppierungen

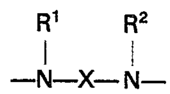 oder 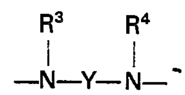

Piperazinylen-Reste treten,

$E^1$ ein Halogenatom oder eine Gruppe

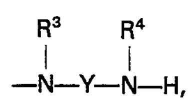 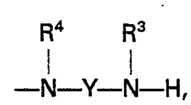

—O—$R^7$, oder —$NR^8R^9$ und

$E^2$ Wasserstoff oder eine Acylgruppe ist, wobei $R^3$, $R^4$, $R^7$, $R^8$ und $R^9$ die oben angegebenen Bedeutungen haben, und mindestens einer der Reste $R^1$, $R^2$ $R^3$, $R^4$, $R^7$, $R^8$ und $R^9$ eine Gruppe der Formel (II) sein muß, mit der Bedingung, daß $R^1$ oder $R^4$ gleich sind, wenn X und Y verschieden sind, und X und Y gleich sind, wenn mindestens einer der Rest $R^1$ oder $R^2$ von $R^3$ und $R^4$ verschieden ist, dadurch gekennzeichnet, daß man in einem inerten, organischen Lösungsmittel entweder

A. ein Dihalogen-bis-triazinyldiamin der Formel (III)

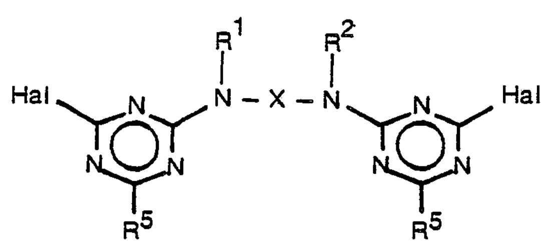 (III)

mit der 0,8- bis 1,4 fach molaren Menge eines Diamins der Formel (IV)

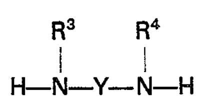 (IV),

wobei in diesen Formeln Hal, $R^1$, $R^2$ $R^3$, $R^4$, $R^5$. X und Y die oben angegebenen Bedeutungen haben, in Gegenwart der—bezogen auf Verbindung (IV)—äquivalenten Menge einer als Halogenwasserstoffakzeptor

dienenden anorganischen Base bei 50 bis 200°C umsetzt, oder

B. ein Poly-bis-triazinylamin der allgemeinen Formel (V)

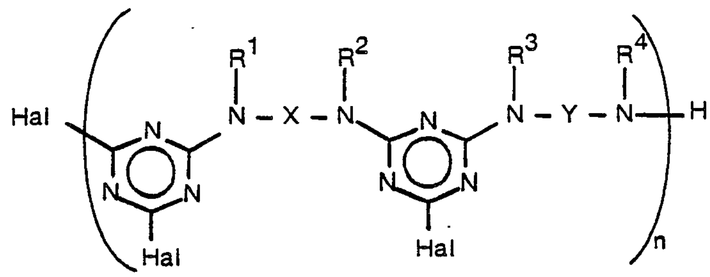

(V)

mit der 0,8- bis 1,4 fach valenten Menge einer Verbindung der Formel (VI)

$$H—R^5 \qquad (VI),$$

wobei in diesen Formeln Hal, $R^1$, $R^2$ $R^3$, $R^4$, $R^5$. X und Y die oben angegebenen Bedeutungen haben, jedoch $R^5$ nicht Hal oder Phenyl ist, in Gegenwart der—bezogen auf Verbindung (VI)—äquivalenten Menge einer anorganischen Base bei 50 bis 200°C zur Reaktion bringt, oder

C. zunächst bei −5 bis 40°C in Gegenwart der äquivalenten Menge eines Halogenwasserstoffakzeptors aus Cyanurhalogenid und die 0,8- bis 1,3 fach molaren Menge einer Verbindung der Formel (VI), wobei jedoch $R^5$ nicht Hal oder Phenyl ist, zu einem Dihalogentriazin der Formel (VII)

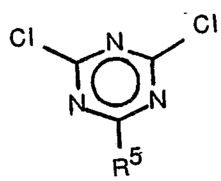

(VII),

umsetzt, dieses anschließend ohne Zwischenisolierung bei 10 bis 70°C in Gegenwart der äquivalenten Menge eines Halogenwasserstoffakzeptors durch Reaktion mit der 0,4- bis 0,7 fach molaren Menge eines Diamins der Formel (VIII)

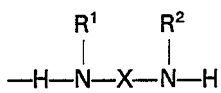

(VIII),

zu einem Dihalogen-bis-triazinyldiamin der Formel (III) derivatisiert, und die so erhaltenen Verbindungen (III) is situ gemäß Verfahren A zu den gewünschten Endprodukten umsetzt, wobei in den Formeln Hal, $R^1$, $R^2$ $R^3$, $R^4$, $R^5$. X und Y die oben angegebenen Bedeutungen haben.

2. Verwendung der Verbindungen nach Anspruch 1 zum Stabilisieren von synthetischen Polymeren.

3. Verwendung nach Anspruch 2, dadurch gekennzeichnet, daß das Polymere ein Polyolefin ist.

4. Verwendung nach Anspruch 2, dadurch gekennzeichnet, daß das Polymere ein Polyacrylat oder Polymethacrylat ist.

5. Verwendung nach Anspruch 2, dadurch gekennzeichnet, daß das Polymere ein Homo- oder Copolymerisat des Styrols ist.

6. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

7. Gegen UV-Zersetzung stabilisierte, synthetische Polymere, in denen 0,01 bis 5 Gewichtseile, bezogen auf Polymeres, eines Stabilisators nach Anspruch 1 enthalten sind.

**Claims for the Contracting States: BE CH DE FR GB IT LI NL SE**

1. A poly-bis-triazinylamine of the general formula (I)

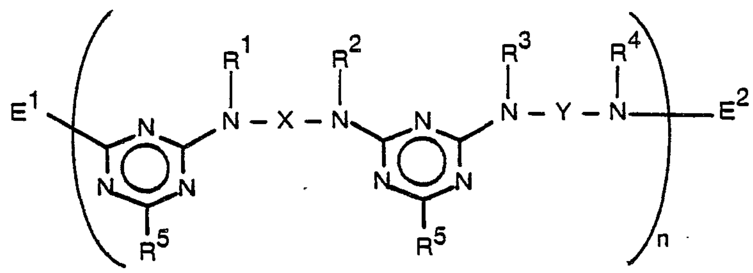

(I)

in which

n is an integer from 1 to 200,

$R^1$ and $R^2$ are identical or different and denote hydrogen, $C_1$ to $C_{37}$-alkyl, cycloalkyl which is unsubstituted or substituted by up to four $C_1$ to $C_4$ alkyl groups, $C_7$ to $C_{14}$ aralkyl or a group of the formula (II)

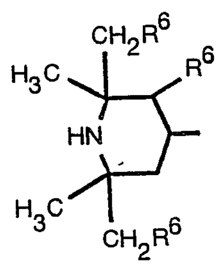

(II)

in which $R^6$ represents hydrogen or $C_1$ to $C_6$ alkyl,

$R^3$ and $R^4$ are identical or different and have the meanings indicated under $R^1$ and $R^2$,

$R^5$ is halogen, phenyl, $-OR^7$ or $-NR^8R^9$, $R^7$ representing hydrogen, $C_1$ to $C_{18}$ alkyl, $C_3$ to $C_{12}$ alkenyl, $C_5$ to $C_{12}$ cycloalkyl, $C_7$ to $C_{18}$ aralkyl, $C_6$ to $C_{10}$ aryl or a radical of the formula (II), and

$R^8$ and $R^9$ being identical or different and denoting hydrogen, $C_1$ to $C_{37}$ alkyl which is unsubstituted or substituted by a hydroxyl group, a $C_1$ to $C_{18}$ alkoxy group or a $C_1$ to $C_4$ dialkylamino group, $C_3$ to $C_{12}$ alkenyl, $C_5$ to $C_{12}$ cycloalkyl, $C_7$ to $C_{18}$ aralkyl, phenyl, naphthyl or a radical of the formula (II), or

$R^8$ and $R^9$, together with the common nitrogen atom, forming a pyrrolidine ring or a piperidine, morpholine or hexamethyleneimine ring which is unsubstituted or substituted by up to four $C_1$ to $C_4$ alkyl groups,

X and Y are identical or different and represent $C_2$ to $C_{18}$ alkylene or $C_2$ to $C_{12}$ bis-(propoxy)-alkylene or $C_6$ to $C_{18}$ monocycloalkylene, dicycloalkylene or tricycloalkylene which are unsubstituted or substituted by up to four methyl groups and in which in the case of the group first mentioned, two C atoms can be replaced by N atoms which can be substituted by propylene groups, or X and Y represent $C_6$ to $C_{18}$ arylene or $C_7$ to $C_{18}$ aralkylene, or piperazinylene radicals are present instead of the groupings

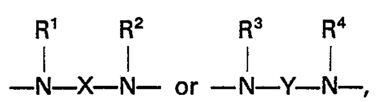

$E^1$ is a halogen atom or a group

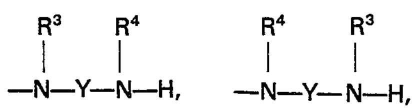

$-O-R^7$ or $-NR^8R^9$ and

$E^2$ is hydrogen or an acyl group, $R^3$, $R^4$, $R^7$, $R^8$ and $R^9$ having the meanings indicated above and at least one of the radicals $R^1$, $R^2$, $R^3$, $R^4$, $R^7$, $R^8$ and $R^9$ being of necessity a group of the formula (II), with the proviso that $R^1$, $R^2$, $R^3$ and $R^4$ are identical, if X and Y are different, and X and Y are identical, if at least one of the radicals $R^1$ or $R^2$ are different from $R^3$ and $R^4$.

2. A process for the preparation of poly-bis-triazinylamines as claimed in claim 1, which comprises reacting, in an inert organic solvent, either

A. a dihalogeno-bis-triazinyldiamine of the formula (III)

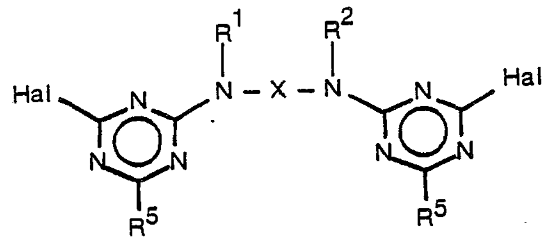

(III)

at 50 to 200°C with a 0.8 to 1.4 molar amount of a diamine of the formula (IV)

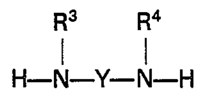

(IV)

Hal, $R^1$, $R^2$, $R^3$, $R^4$, $R^5$, X and Y in these formulae having the meanings indicated in claim 1, in the presence of an equivalent amount — relative to the compound (IV) — of an inorganic base which acts as a hydrogen halide acceptor, or

B. a poly-bis-triazinylamine of the general formula (V)

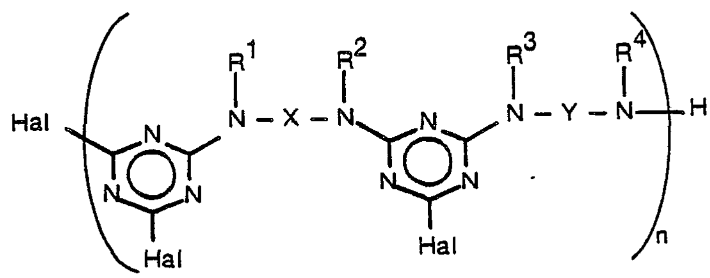

(V)

at 50 to 200°C with a 0.8 to 1.4 equivalent amount of a compound of the formula (VI)

$$H—R^5 \quad (VI)$$

Hal, $R^1$, $R^2$, $R^3$, $R^4$, $R^5$, X and Y in these formulae having the meanings indicated in claim 1, but $R^5$ not being Hal or phenyl, in the presence of an equivalent amount — relative to the compound (VI) — of an inorganic base, or

C. a cyanuric halide and a 0.8 to 1.3 molar amount of a compound of the formula (VI) in which, however, $R^5$ is not Hal or phenyl, initially at −5 to 40°C and in the presence of an equivalent amount of a hydrogen halide acceptor, to give a dihalogenotriazine of the formula (VII)

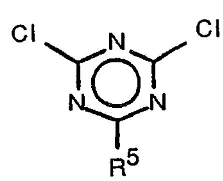

(VII),

then, without intermediate isolation of the latter, deriving from it, at 10 to 70°C and in the presence of an equivalent amount of a hydrogen halide acceptor by reaction with a 0.4 to 0.7 molar amount of a diamine of the formula (VIII)

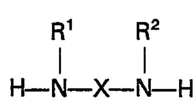

(VIII)

a dihalogeno-bis-triazinyldiamine of the formula (III) and reacting the compounds (III) thus obtained, in situ

in accordance with process A, to give the desired end products, Hal, $R^1$, $R^2$, $R^3$, $R^4$, $R^5$, X and Y in the formulae having the meanings indicated in claim 1.

3. Use of the compounds according to claim 1 for the stabilization of synthetic polymers.

4. The use as claimed in claim 3, wherein the polymer is a polyolefin.

5. The use as claimed in claim 3, wherein the polymer is a polyacrylate or a polymethacrylate.

6. The use as claimed in claim 3, wherein the polymer is a homo- or copolymer of styrene.

7. A process for stabilizing synthetic polymers against the harmful effect of light, which comprises adding to the polymers, if appropriate in addition to hitherto known substances having a stabilizing action, 0.01 to 5 parts by weight, relative to the polymer, of a stabilizer as claimed in claim 1.

8. Synthetic polymers which have been stabilized against decomposition by UV light and which contain 0.01 to 5 parts by weight, relative to the polymer, of a stabilizer as claimed in claim 1.

**Claims for the Contracting State: AT**

1. A process for the preparation of poly-bis-triazinylamines of the general formula (I)

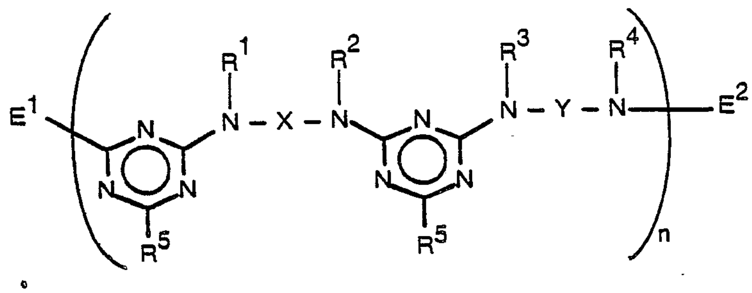

(I)

in which

n is an integer from 1 to 200,

$R^1$ and $R^2$ are identical or different and denote hydrogen, $C_1$ to $C_{37}$ alkyl, cycloalkyl which is unsubstituted or substituted by up to four $C_1$ to $C_4$ alkyl groups, $C_7$ to $C_{14}$ aralkyl or a group of the formula (II)

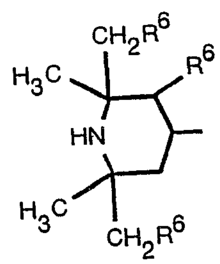

(II)

in which $R^6$ represents hydrogen or $C_1$ to $C_6$ alkyl,

$R^3$ and $R^4$ are identical or different and have the meanings indicated under $R^1$ and $R^2$,

$R^5$ is halogen, phenyl, $—OR^7$ or $—NR^8R^9$, $R^7$ representing hydrogen, $C_1$ to $C_{18}$ alkyl, $C_3$ to $C_{12}$ alkenyl, $C_5$ to $C_{12}$ cycloalkyl, $C_7$ to $C_{18}$ aralkyl, $C_6$ to $C_{10}$ aryl or a radical of the formula (II), and

$R^8$ and $R^9$ being identical or different and denoting hydrogen, $C_1$ to $C_{37}$ alkyl which is unsubstituted or substituted by a hydroxyl group, a $C_1$ to $C_{18}$ alkoxy group or a $C_1$ to $C_4$ dialkylamino group, $C_3$ to $C_{12}$ alkenyl, $C_5$ to $C_{12}$ cycloalkyl, $C_7$ to $C_{18}$ aralkyl, phenyl, naphthyl or a radical of the formula (II), or

$R^8$ and $R^9$, together with the common nitrogen atom, forming a pyrrolidine ring or a piperidine, morpholine or hexamethyleneimine ring which is unsubstituted or substituted by up to four $C_1$ to $C_4$ alkyl groups,

X and Y are identical or different and represent $C_2$ to $C_{18}$ alkylene or $C_2$ to $C_{12}$ bis-(propoxy)-alkylene or $C_6$ to $C_{18}$ monocycloalkylene, dicycloalkylene or tricycloalkylene which are unsubstituted or substituted by up to four methyl groups and in which in the case of the group first mentioned, two C atoms can be replaced by N atoms which can be substituted by propylene groups, or X and Y represent $C_6$ to $C_{18}$ arylene or $C_7$ to $C_{18}$ aralkylene, or piperazinylene radicals are present instead of the groupings

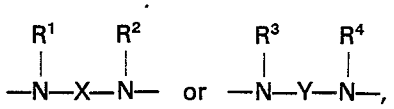

$E^1$ is a halogen atom or a group

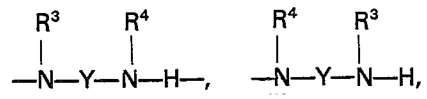

—O—$R^7$ or —$NR^8R^9$ and

$E^2$ is hydrogen or an acyl group, $R^3$, $R^4$, $R^7$, $R^8$ and $R^9$ having the meanings indicated above and at least one of the radicals $R^1$, $R^2$, $R^3$, $R^4$, $R^7$, $R^8$ and $R^9$ being of necessity a group of the formula (II), with the proviso that $R^1$, $R^2$, $R^3$ and $R^4$ are identical, if X and Y are different, and X and Y are identical, if at least one of the radicals $R^1$ or $R^2$ are different from $R^3$ and $R^4$, which comprises reacting, in an inert organic solvent, either

A. a dihalogeno-bis-triazinyldiamine of the formula (III)

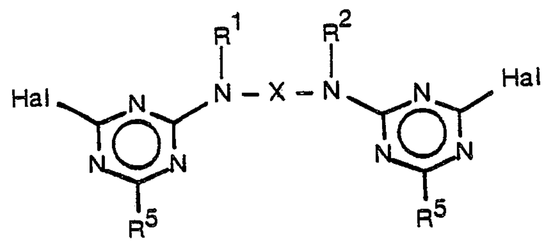

(III)

at 50 to 200°C with a 0.8 to 1.4 molar amount of a diamine of the formula (IV)

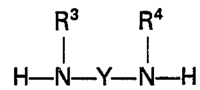

(IV)

Hal, $R^1$, $R^2$, $R^3$, $R^4$, $R^5$, X and Y in these formulae having the meanings indicated above, in the presence of an equivalent amount — relative to the compound (IV) — of an inorganic base which acts as a hydrogen halide acceptor, or

B. a poly-bis-triazinylamine of the general formula (V)

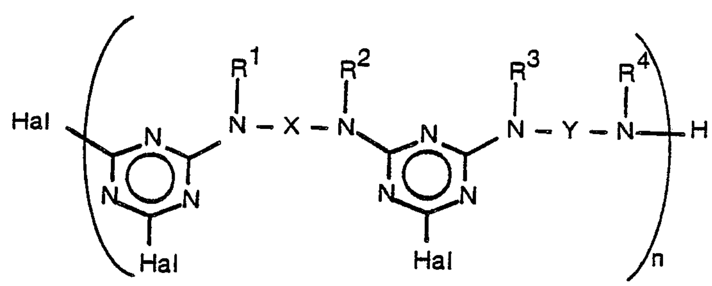

(V)

at 50 to 200°C with a 0.8 to 1.4 equivalent amount of a compound of the formula (VI)

$$H—R^5 \qquad (VI)$$

Hal, $R^1$, $R^2$, $R^3$, $R^4$, $R^5$, X and Y in these formulae having the meanings indicated in claim 1, but $R^5$ not being Hal or phenyl, in the presence of an equivalent amount — relative of the compound (VI) — of an inorganic base, or

C. a cyanuric halide and a 0.8 to 1.3 molar amount of a compound of the formula (VI) in which, however, $R^5$ is not Hal or phenyl, initially at −5 to 40°C and in the presence of an equivalent amount of a hydrogen halide acceptor, to give a dihalogenotriazine of the formula (VII)

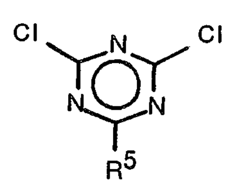

(VII),

then, without intermediate isolation of the latter, deriving from it, at 10 to 70°C and in the presence of an equivalent amount of a hydrogen halide acceptor, by reaction with a 0.4 to 0.7 molar amount of a diamine of the formula (VIII)

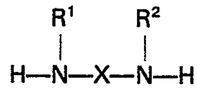

(VIII)

a dihalogeno-bis-triazinyldiamine of the formula (III) and reacting the compounds (III) thus obtained, in situ in accordance with process A, to give the desired end products, Hal, $R^1$, $R^2$, $R^3$, $R^4$, $R^5$, X and Y in the formulae having the meanings indicated above.

2. Use of the compounds according to claim 1 for the stabilization of synthetic polymers.

3. The use as claimed in claim 2, wherein the polymer is a polyolefin.

4. The use as claimed in claim 2, wherein the polymer is a polyacrylate or a polymethacrylate.

5. The use as claimed in claim 2, wherein the polymer is a homo- or copolymer of styrene.

6. A process for stabilizing synthetic polymers against the harmful effect of light, which comprises adding to the polymers, if appropriate in addition to hitherto known substances having a stabilizing action, 0.01 to 5 parts by weight, relative to the polymer, of a stabilizer as claimed in claim 1.

7. Synthetic polymers which have been stabilized against decomposition by UV light and which contain 0.01 to 5 parts by weight, relative to the polymer, of a stabilizer as claimed in claim 1.

**Revendications pour les Etats contractants: BE CH DE FR GB IT LI NL SE**

1. Poly-(bis-triazinylamines) répondant à la formule générale I

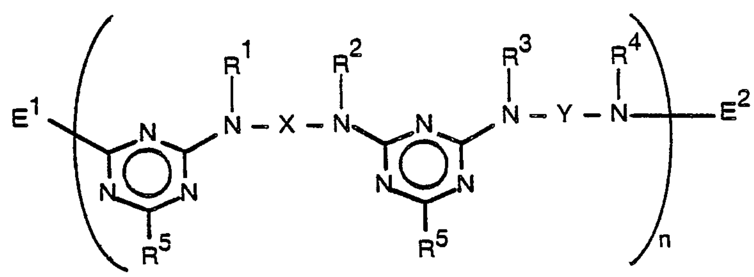

(I)

dans laquelle:

n représente un nombre entier de 1 à 200,

$R^1$ et $R^2$ sont identiques ou différents et représentent chacun l'hydrogène, un alkyle en $C_1$—$C_{37}$, un cycloalkyle non substitué ou porteur d'au plus quatre radicaux alkyles en $C_1$—$C_4$, un aralkyle en $C_7$—$C_{14}$ ou un radical de formule II:

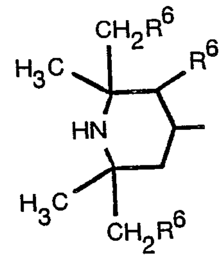

(II)

dans lequel $R^6$ représente l'hydrogène ou un alkyle en $C_1$—$C_6$,

$R^3$ et $R^4$ sont identiques ou différents et ont les significations qui ont été indiquées pour $R^1$ et $R^2$,

$R^5$ représente un halogène, un phényle, un radical —$OR^7$ ou un radical —$NR^8R^9$, dans lesquels $R^7$ représente l'hydrogène, un alkyle en $C_1$—$C_{18}$, un alcényle en $C_3$—$C_{12}$, un cycloalkyle en $C_5$—$C_{12}$, un aralkyle en $C_7$—$C_{18}$, un aryle en $C_6$—$C_{10}$ ou un radical de formule II, et

$R^8$ et $R^9$ sont identiques ou différents et représentent chacun l'hydrogène, un alkyle en $C_1$—$C_{37}$ non substitué ou porteur d'un hydroxy, d'un alcoxy en $C_1$—$C_{18}$ ou d'un dialkylamino dont chacun des alkyles contient de 1 à 4 atomes de carbone, un alcényle en $C_3$—$C_{12}$, un cycloalkyle en $C_5$—$C_{12}$, un aralkyle en $C_7$—$C_{18}$, un phényle, un naphtyle ou un radical de formule II, ou encore

$R^8$ et $R^9$ forment ensemble et avec l'atome d'azote qui les porte un cycle de pyrrolidine ou un cycle de pipéridine, de morpholine ou de perhydro-azépine non substitué ou porteur d'au plus quatre radicaux alkyles en $C_1$—$C_4$,

X et Y sont identiques ou différents et représentent chacun un alkylène en $C_2$—$C_{18}$, un bis-(propylène-oxy)-alkylène en $C_2$—$C_{12}$, un radical mono-, di- ou tri-cycloalkylène non substitué ou porteur d'au plus quatre radicaux méthyles, dans lequel, pour le premier cité, deux atomes de carbone peuvent être remplacés par des atomes d'azote éventuellement porteurs de radicaux propylènes, un arylène en $C_6$—$C_{18}$ ou un aralkylène en $C_7$—$C_{18}$, ou encore les groupements:

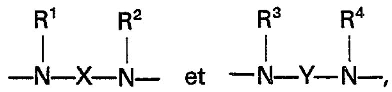

peuvent être remplacés par des radicaux pipérazinylènes,

$E^1$ représente un atome d'halogène ou un radical

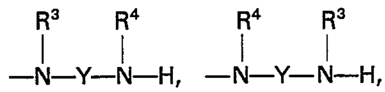

$-O-R^7$ ou $-NR^8R^9$ et

$E^2$ représente l'hydrogène ou un radical acyle, les symboles $R^3$, $R^4$, $R^7$, $R^8$ et $R^9$ ayant les significations indiquées ci-dessus,

avec la condition qu'au moins l'un des symboles $R^1$, $R^2$, $R^3$, $R^4$, $R^7$, $R^8$ et $R^9$ représente un radical de formule II,

et la condition que $R^1$ à $R^3$ soient identiques lorsque X et Y sont différents, et que X et Y soient identiques lorsqu'au moins l'un des radicaux $R^1$ et $R^2$ est différent de $R^3$ et $R^4$.

2. Procédé de préparation de poly-(bis-triazinylamines) selon la revendication 1, procédé caractérisé en ce que, en opérant dans un solvant inerte, ou bien:

A. on fait réagir, à une température de 50 à 200°C, 1 mol d'une dihalogéno-bis-triazinyl-diamine de formule III:

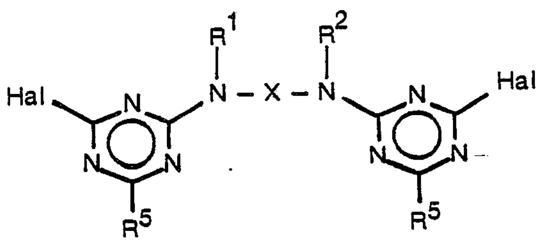

(III)

avec de 0,8 à 1,4 mol d'une diamine de formule IV:

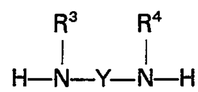

(IV),

formules dans lesquelles Hal, $R^1$, $R^2$, $R^3$, $R^4$, $R^5$, X et Y ont les significations données à la revendication 1, en présence d'une quantité équivalente, par rapport au composé (IV), d'une base minérale servant d'accepteur d'acide halohydrique, ou bien

B. on fait réagir, à une température de 50 à 200°C, une poly-(bis-triazinylamine) de formule générale V:

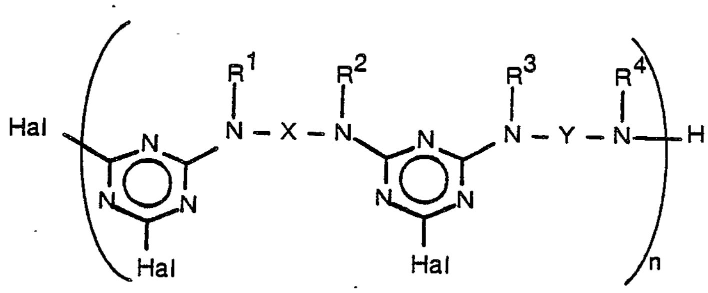

(V)

avec de 0,8 à 1,4 fois la quantité équivalente d'un composé de formule VI:

$$H-R^5 \qquad (VI),$$

formules dans lesquelles Hal, $R^1$, $R^2$, $R^3$, $R^4$, $R^5$, X et Y ont les significations données à la revendication 1 mais $R^5$ ne peut être ni un halogène ni un phényle, en présence d'une quantité équivalente, par rapport au composé (VI), d'une base minérale, ou bien,

C. on commence par faire réagir, à une température de −5 à 40°C, en présence d'une quantité équivalente d'un accepteur d'acide halohydrique, 1 mol d'un halogénure de cyanuryle et de 0,8 à 1,3 mol d'un composé de formule VI dans lequel toutefois $R^5$ ne peut être ni un halogène ni un phényle, réaction qui conduit à une dihalogénotriazine de formule VII:

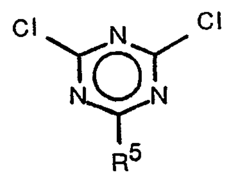

(VII),

on fait ensuite réagir 1 mol de celle-ci, sans l'isoler intermédiairement, à une température de 10 à 70°C, en présence d'une quantité équivalente d'un accepteur d'acide halohydrique, avec de 0,4 à 0,7 mol d'une diamine de formule VIII:

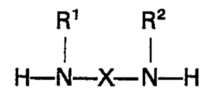

(VIII),

de manière à obtenir une dihalogéno-bis-triazinyl-diamine de formule III, et on fait réagir le composé (III) ainsi obtenu, in situ, selon le procédé A, de manière à obtenir les produits finals cherchés (dans les formules précédentes, Hal, $R^1$, $R^2$, $R^3$, $R^4$, $R^5$, X et Y ont les significations qui ont été données à la revendication 1).

3. Application des composés selon la revendication 1 à la stabilisation de polymères synthétiques.

4. Application selon la revendication 3, caractérisée en ce que le polymère est une polyoléfine.

5. Application selon la revendication 3, caractérisée en ce que le polymère est un polyacrylate ou un polyméthacrylate.

6. Application selon la revendication 3, caractérisée en ce que le polymère est un homopolymère ou un copolymère du styrène.

7. Procédé pour stabiliser des polymères synthétiques contre les effets destructeurs de la lumière, procédé caractérisé en ce qu'on ajoute aux polymères, éventuellement en plus de composés à action stabilisante connus jusqu'à présent, de 0,01 à 5% en poids, par rapport au polymère, d'un stabilisant selon la revendication 1.

8. Polymères synthétiques stabilisés contre la dégradation par les rayons UV, polymères qui contiennent de 0,01 à 5% en poids, par rapport au polymère, d'un stabilisant selon la revendication 1.

**Revendications pour l'Etat contractant: AT**

1. Procédé de préparation de poly-(bis-triazinylamines) répondant à la formule générale I:

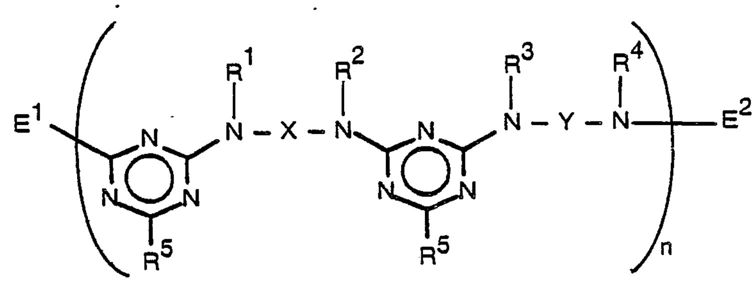

(I)

dans laquelle:

n représente un nombre entier de 1 à 200,

$R^1$ et $R^2$ sont identiques ou différents et représentent chacun l'hydrogène, un alkyle en $C_1$—$C_{37}$, un cycloalkyle non substitué ou porteur d'au plus quatre radicaux alkyles en $C_1$—$C_4$, un aralkyle en $C_7$—$C_{14}$ ou un radical de formule II:

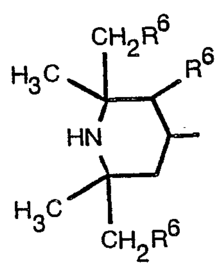

(II),

dans lequel $R^6$ représente l'hydrogène ou un alkyle en $C_1$—$C_6$,

$R^3$ et $R^4$ sont identiques ou différents et ont les significations qui ont été indiquées pour $R^1$ et $R^2$,

$R^5$ représente un halogène, un phényle, un radical —$OR^7$ ou un radical —$NR^8R^9$, dans lesquels $R^7$ représente l'hydrogène, un alkyle en $C_1$—$C_{18}$, un alcényle en $C_3$—$C_{12}$, un cycloalkyle en $C_5$—$C_{12}$, un aralkyle en $C_7$—$C_{18}$, un aryle en $C_6$—$C_{10}$ ou un radical de formule II, et

$R^8$ et $R^9$ sont identiques ou différents et représentent chacun l'hydrogène, un alkyle en $C_1$—$C_{37}$ non substitué ou porteur d'un hydroxy, d'un alcoxy en $C_1$—$C_{18}$ ou d'un dialkylamino dont chacun des alkyles contient de 1 à 4 atomes de carbone, un alcényle en $C_3$—$C_{12}$, un cycloalkyle en $C_5$—$C_{12}$, un aralkyle en $C_7$—$C_{18}$, un phényle, un naphtyle ou un radical de formule II, ou encore

$R^8$ et $R^9$ forment ensemble et avec l'atome d'azote qui les porte un cycle de pyrrolidine ou un cycle de pipéridine, de morpholine ou de perhydro-azépine non substitué ou porteur d'au plus quatre radicaux alkyles en $C_1$—$C_4$,

X et Y sont identiques ou différents et représentent chacun un alkylène en $C_2$—$C_{18}$, un bis-(propylène-oxy)-alkylène en $C_2$—$C_{12}$, un radical mono-, di- ou tri-cycloalkylène non substitué ou porteur d'au plus quatre radicaux méthyles, dans lequel, pour le premier cité, deux atomes de carbone peuvent être remplacés par des atomes d'azote éventuellement porteurs de radicaux propylènes, un arylène en $C_6$—$C_{18}$ ou un aralkylène en $C_7$—$C_{18}$, ou encore les groupements:

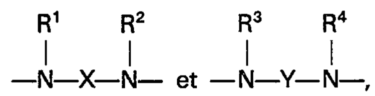

peuvent être remplacés par des radicaux pipérazinylènes,

$E^1$ représente un atome d'halogène ou un radical

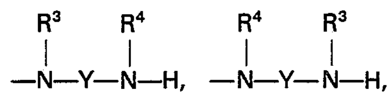

—$OR^7$ ou —$NR^8R^9$ et

$E^2$ représente l'hydrogène ou un radical acyle, les symboles $R^3$, $R^4$, $R^7$, $R^8$ et $R^9$ ayant les significations indiquées ci-dessus,

avec la condition qu'au moins l'un des symboles $R^1$, $R^2$, $R^3$, $R^4$, $R^7$, $R^8$ et $R^9$ représente un radical de formule II,

et la condition que $R^1$ à $R^4$ soient identiques lorsque X et Y sont différents, et que X et Y soient identiques lorsqu'au moins l'un des radicaux $R^1$ et $R^2$ est différent de $R^3$ et $R^4$,

procédé caractérisé en ce que, en opérant dans un solvant organique inerte, ou bien

A. on fait réagir, à une température de 50 à 200°C, 1 mol d'une dihalogéno-bis-triazinyl-diamine de formule III:

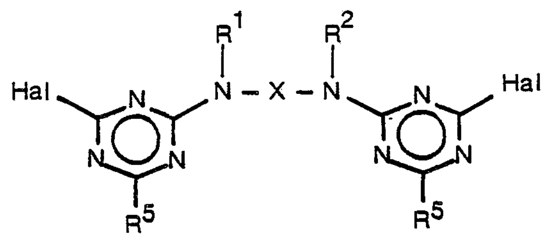

(III)

avec de 0,8 à 1,4 mol d'une diamine de formule IV:

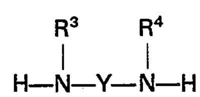

(IV),

formules dans lesquelles Hal, $R^1$, $R^2$, $R^3$, $R^4$, $R^5$, X et Y ont les significations données ci-dessus, en présence d'une quantité équivalente, par rapport au composé (IV), d'une base minérale servant d'accepteur d'acide halohydrique, ou bien

B. on fait réagir, à une température de 50 à 200°C, une poly-(bis-triazinylamine) de formule générale V:

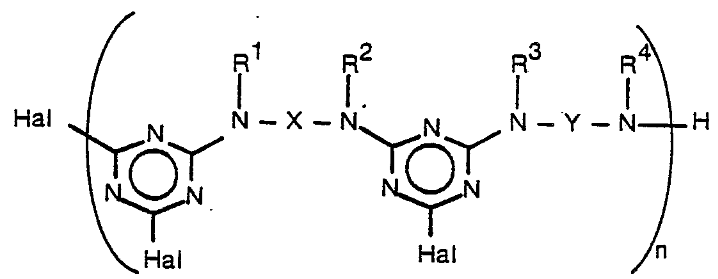

(V)

avec de 0,8 à 1,4 fois la quantité équivalente d'un composé de formule VI:

$$H—R^5 \quad (VI),$$

formules dans lesquelles Hal, $R^1$, $R^2$, $R^3$, $R^4$, $R^5$, X et Y ont les significations données ci-dessus mais $R^5$ ne peut être ni un halogène ni un phényle, en présence d'une quantité équivalente, par rapport au composé (VI), d'une base minérale, ou bien,

C. on commence par faire réagir, à une température de −5 à 40°C, en présence d'une quantité équivalente d'un accepteur d'acide halohydrique, 1 mol d'un halogénure de cyanuryle et de 0,8 à 1,3 mol d'un composé de formule VI dans lequel toutefois $R^5$ ne peut être ni un halogène ni un phényle, réaction qui conduit à une dihalogénotriazine de formule VII:

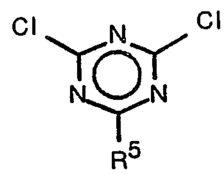

(VII),

on fait ensuite réagir 1 mol de celle-ci, sans l'isoler intermédiairement, à une température de 10 à 70°C, en présence d'une quantité équivalente d'un accepteur d'acide halohydrique, avec de 0,4 à 0,7 mol d'une diamine de formule VIII:

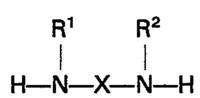

(VIII),

de manière à obtenir une dihalogéno-bis-triazinyl-diamine de formule III, et on fait réagir le composé (III) ainsi obtenu, in situ, selon le procédé A, de manière à obtenir les produits finals cherchés (dans les formules précédentes, Hal, $R^1$, $R^2$, $R^3$, $R^4$, $R^5$, X et Y ont les significations qui ont été données ci-dessus).

2. Application des composés selon la revendication 1 à la stabilisation de polymères synthétiques.

3. Application selon la revendication 2, caractérisée en ce que le polymère est une polyoléfine.

4. Application selon la revendication 2, caractérisée en ce que le polymère est un polyacrylate ou un polyméthacrylate.

5. Application selon la revendication 2, caractérisée en ce que le polymère est un homopolymère ou un copolymère du styrène.

6. Procédé pour stabiliser des polymères synthétiques contre les effets destructeurs de la lumière, procédé caractérisé en ce qu'on ajoute aux polymères, éventuellement en plus de composés à action stabilisante connus jusqu'à présent, de 0,01 à 5% en poids, par rapport au polymère, d'un stabilisant selon la revendication 1.

7. Polymères synthétiques stabilisés contre la dégradation par les rayons UV, polymères qui contiennent de 0,01 à 5% en poids, par rapport au polymère, d'un stabilisant selon la revendication 1.